# EUROPEAN PATENT SPECIFICATION

(11) **EP 2 566 965 B1**
(45) Date of publication and mention of the grant of the patent: **28.04.2021**
(21) Application number: 11717668.5
(22) Date of filing: 06.05.2011
(51) Int. Cl.: C12N 15/113, C07K 14/705, A61K 38/17

(54) **ANTAGONISTS OF SEMA3E/PLEXIND1 INTERACTION AS ANTI-CANCER AGENTS**
ANTAGONISTEN DER SEMA3E/PLEXIND1-INTERAKTION ALS ANTIKREBSWIRKSTOFF
ANTAGONISTES DE L'INTERACTION DE SEMA3E/PLEXIND1 COMME AGENTS ANTI-CANCER

(30) Priority: 06.05.2010 EP 10305480
(43) Date of publication of application: 13.03.2013
(73) Proprietor: Netris Pharma, 69008 Lyon (FR); Centre National de la Recherche Scientifique (C.N.R.S.), 75016 Paris (FR); Universite d'Aix-Marseille, 13007 Marseille (FR)
(72) Inventor: ROYET, Amélie, F-69440 Mornant (FR); MANN, Fanny, F-13600 La Ciotat (FR); CHAUVET, Sophie, F-13005 Marseille (FR); LUCHINO, Jonathan, F-13004 Marseille (FR)
(74) Representative: Lavoix
(86) International application number: PCT/EP2011/057347
(87) International publication number: WO 2011/138449

(56) References cited:
- WO-A2-01/14420
- ROODINK ILSE ET AL: "PLEXIN D1 EXPRESSION IS INDUCED ON TUMOR VASCULATURE AND TUMOR CELLS: A NOVEL TARGET FOR DIAGNOSIS AND THERAPY?", CANCER RESEARCH, AMERICAN ASSOCIATION FOR CANCER REREARCH, US LNKD- DOI:10.1158/0008-5472.CAN-04-4366, vol. 65, no. 18, 1 September 2005 (2005-09-01), pages 8317-8323, XP009076630, ISSN: 0008-5472
- ROODINK ILSE ET AL: "Semaphorin 3E Expression Correlates Inversely with Plexin D1 During Tumor Progression", AMERICAN JOURNAL OF PATHOLOGY, AMERICAN SOCIETY FOR INVESTIGATIVE PATHOLOGY, US LNKD- DOI:10.2353/AJPATH.2008.080136, vol. 173, no. 6, 1 December 2008 (2008-12-01), pages 1873-1881, XP009114920, ISSN: 0002-9440
- SAKURAI ATSUKO ET AL: "Semaphorin 3E initiates antiangiogenic signaling through plexin D1 by regulating Arf6 and R-Ras.", MOLECULAR AND CELLULAR BIOLOGY JUN 2010 LNKD- PUBMED:20385769, vol. 30, no. 12, 12 April 2010 (2010-04-12), pages 3086-3098, XP009137802, ISSN: 1098-5549
- MORIYA JUNJI ET AL: "Semaphorin3E is a Novel Angiogenic Regulator", CIRCULATION, LIPPINCOTT WILLIAMS & WILKINS, US, vol. 118, no. 18, suppl 2, 1 October 2008 (2008-10-01), page S459, XP009114917, ISSN: 0009-7322
- GUIDO SERINI ET AL: "Semaphorins and tumor angiogenesis", ANGIOGENESIS, KLUWER ACADEMIC PUBLISHERS, DO, vol. 12, no. 2, 6 March 2009 (2009-03-06), pages 187-193, XP019668700, ISSN: 1573-7209
- CHRISTENSEN CLAUS ET AL: "Proteolytic processing converts the repelling signal Sema3E into an inducer of invasive growth and lung metastasis", CANCER RESEARCH, AMERICAN ASSOCIATION FOR CANCER REREARCH, US, [Online] vol. 65, no. 14, 15 July 2005 (2005-07-15) , pages 6167-6177, XP002522809, ISSN: 0008-5472, DOI: DOI:10.1158/0008-5472.CAN-04-4309 Retrieved from the Internet: URL:http://cancerres.aacrjournals.org/cgi/ content/full/65/14/6167> [retrieved on 2011-05-26]
- CASAZZA ANDREA ET AL: "Sema3E-Plexin D1 signaling drives human cancer cell invasiveness and metastatic spreading in mice", JOURNAL OF CLINICAL INVESTIGATION, AMERICAN SOCIETY FOR CLINICAL INVESTIGATION, US LNKD- DOI:10.1172/JCI42118, vol. 120, no. 8, 2 August 2010 (2010-08-02), pages 2684-2698, XP009137280, ISSN: 0021-9738
- BLANC V ET AL: "A Role for Class 3 Semaphorins in Prostate Cancer", PROSTATE, vol. 71, no. 6, 14 October 2010 (2010-10-14), pages 649-658, XP002638766, online
- TILMAN SCHNEIDER-POETSCH ET AL: "Inhibition of eukaryotic translation elongation by cycloheximide and lactimidomycin", NATURE CHEMICAL BIOLOGY, vol. 6, no. 3, 31 January 2010 (2010-01-31), pages 209-217, XP055389563, Basingstoke ISSN: 1552-4450, DOI: 10.1038/nchembio.304

### SEQUENCE LISTING

<110> NETRIS PHARMA CNRS (Paris) UNIVERSITE DE LA MEDITERRANEE
<120> Antagonists of Sema3E/PlexinDl interaction as anti-cancer agents
<130> BET10L0712
<160> 27
<170> PatentIn version 3.4
<210> 1
   <211> 471
   <212> PRT
   <213> homo sapiens
<400> 1
   Asn Asn Phe Ala Leu Asp Gly Ala Ala Gly Thr Val Tyr Leu Ala Ala
   1               5                   10                  15
   Val Asn Arg Leu Tyr Gln Leu Ser Gly Ala Asn Leu Ser Leu Glu Ala
               20                  25                  30
   Glu Ala Ala Val Gly Pro Val Pro Asp Ser Pro Leu Cys His Ala Pro
           35                  40                  45
   Gln Leu Pro Gln Ala Ser Cys Glu His Pro Arg Arg Leu Thr Asp Asn
       50                  55                  60
   Tyr Asn Lys Ile Leu Gln Leu Asp Pro Gly Gln Gly Leu Val Val Val
   65                  70                  75                  80
   Cys Gly Ser Ile Tyr Gln Gly Phe Cys Gln Leu Arg Arg Arg Gly Asn
                   85                  90                  95
   Ile Ser Ala Val Ala Val Arg Phe Pro Pro Ala Ala Pro Pro Ala Glu
               100                 105                 110
   Pro Val Thr Val Phe Pro Ser Met Leu Asn Val Ala Ala Asn His Pro
           115                 120                 125
   Asn Ala Ser Thr Val Gly Leu Val Leu Pro Pro Ala Ala Gly Ala Gly
       130                 135                 140
   Gly Ser Arg Leu Leu Val Gly Ala Thr Tyr Thr Gly Tyr Gly Ser Ser
   145                 150                 155                 160
   Phe Phe Pro Arg Asn Arg Ser Leu Glu Asp His Arg Phe Glu Asn Thr
                   165                 170                 175
   Pro Glu Ile Ala Ile Arg Ser Leu Asp Thr Arg Gly Asp Leu Ala Lys
               180                 185                 190
   Leu Phe Thr Phe Asp Leu Asn Pro Ser Asp Asp Asn Ile Leu Lys Ile
           195                 200                 205
   Lys Gln Gly Ala Lys Glu Gln His Lys Leu Gly Phe Val Ser Ala Phe
       210                 215                 220
   Leu His Pro Ser Asp Pro Pro Pro Gly Ala Gln Ser Tyr Ala Tyr Leu
   225                 230                 235                 240
   Ala Leu Asn Ser Glu Ala Arg Ala Gly Asp Lys Glu Ser Gln Ala Arg
                   245                 250                 255
   Ser Leu Leu Ala Arg Ile Cys Leu Pro His Gly Ala Gly Gly Asp Ala
               260                 265                 270
   Lys Lys Leu Thr Glu Ser Tyr Ile Gln Leu Gly Leu Gln Cys Ala Gly
           275                 280                 285
   Gly Ala Gly Arg Gly Asp Leu Tyr Ser Arg Leu Val Ser Val Phe Pro
       290                 295                 300
   Ala Arg Glu Arg Leu Phe Ala Val Phe Glu Arg Pro Gln Gly Ser Pro
   305                 310                 315                 320
   Ala Ala Arg Ala Ala Pro Ala Ala Leu Cys Ala Phe Arg Phe Ala Asp
                   325                 330                 335
   Val Arg Ala Ala Ile Arg Ala Ala Arg Thr Ala Cys Phe Val Glu Pro
               340                 345                 350
   Ala Pro Asp Val Val Ala Val Leu Asp Ser Val Val Gln Gly Thr Gly
           355                 360                 365
   Pro Ala Cys Glu Arg Lys Leu Asn Ile Gln Leu Gln Pro Glu Gln Leu
       370                 375                 380
   Asp Cys Gly Ala Ala His Leu Gln His Pro Leu Ser Ile Leu Gln Pro
   385                 390                 395                 400
   Leu Lys Ala Thr Pro Val Phe Arg Ala Pro Gly Leu Thr Ser Val Ala
                   405                 410                 415
   Val Ala Ser Val Asn Asn Tyr Thr Ala Val Phe Leu Gly Thr Val Asn
               420                 425                 430
   Gly Arg Leu Leu Lys Ile Asn Leu Asn Glu Ser Met Gln Val Val Ser
               435                 440                 445
   Arg Arg Val Val Thr Val Ala Tyr Gly Glu Pro Val His His Val Met
       450                 455                 460
   Gln Phe Asp Pro Ala Asp Ser
   465                 470
<210> 2
   <211> 1413
   <212> DNA
   <213> homo sapiens
<400> 2
   aacaacttcg ccctggacgg cgcggcgggg accgtgtacc tggcggccgt caaccgcctc     60
   tatcagctgt cgggcgccaa cctgagcctg gaggccgagg cggccgtggg cccggtgccc    120
   gacagcccgc tgtgtcacgc tccgcagctg ccgcaggcct cgtgcgagca cccgcggcgc    180
   ctcacggaca actacaacaa gatcctgcag ctggaccccg gccagggcct ggtagtcgtg    240
   tgcgggtcca tctaccaggg cttctgccag ctgcggcgcc ggggcaacat ctcggccgtg    300
   gccgtgcgct tcccgcccgc cgcgccgccc gccgagcccg tcacggtgtt ccccagcatg    360
   ctgaacgtgg cggccaacca cccgaacgcg tccaccgtgg ggctagttct gcctcccgcc    420
   gcgggcgcgg ggggcagccg cctgctcgtg ggcgccacgt acaccggtta cggcagctcc    480
   ttcttcccgc gcaaccgcag cctggaggac caccgcttcg agaacacgcc cgagatcgcc    540
   atccgctccc tggacacgcg cggcgacctg gccaagctct tcaccttcga cctcaacccc    600
   tccgacgaca acatcctcaa gatcaagcag ggcgccaagg agcagcacaa gctgggcttc    660
   gtgagcgcct tcctgcaccc gtccgacccg ccgccgggtg cacagtccta cgcgtacctg    720
   gcgctcaaca gcgaggcgcg cgcgggcgac aaggagagcc aggcgcggag cctgctggcg    780
   cgcatctgcc tgccccacgg cgccggcggc gacgccaaga agctcaccga gtcctacatc    840
   cagttgggct tgcagtgcgc gggcggcgcg ggccgcggcg acctctacag ccgcctggtg    900
   tcggtcttcc cagcccggga gcggctcttt gctgtcttcg agcggcccca ggggtccccc    960
   gcggcccgcg ctgctccggc cgcactctgc gccttccgct tcgccgacgt gcgagccgcc   1020
   atccgagctg cgcgcaccgc ctgcttcgtg gaaccggcgc ccgacgtggt ggcggtgctc   1080
   gacagcgtgg tgcagggcac gggaccggcc tgcgagcgca agctcaacat ccagctccag   1140
   ccagagcagc tggactgtgg agctgctcac ctgcagcacc cgctgtccat cctgcagccc   1200
   ctgaaggcca cgcccgtgtt ccgcgccccg ggcctcacct ccgtggccgt ggccagcgtc   1260
   aacaactaca cagcggtctt cctgggcacg gtcaacggga ggcttctcaa gatcaacctg   1320
   aacgagagca tgcaggtggt gagcaggcgg gtggtgactg tggcctatgg ggagcccgtg   1380
   caccatgtca tgcagtttga cccagcagac tcc                                1413
<210> 3
   <211> 227
   <212> PRT
   <213> homo sapiens
<400> 3
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Glu Glu Met Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
                           225
<210> 4
   <211> 22
   <212> DNA
   <213> artificial sequence
<220>
   <223> primer
<400> 4
   tgtatgggcg aataactcac tg 22
<210> 5
   <211> 22
   <212> DNA
   <213> artificial sequence
<220>
   <223> primer
<400> 5
   tcattccaaa cacagaggtg ac 22
<210> 6
   <211> 56
   <212> DNA
   <213> artificial sequence
<220>
   <223> primer
<400> 6
   ggggaccact ttgtacaaga aagctgggtc ctaggagagc agcgtgtgcc tgggca 56
<210> 7
   <211> 53
   <212> DNA
   <213> artificial sequence
<220>
   <223> primer
<400> 7
   ggggacaagt ttgtacaaaa agcaggcttc gcgaacccct cctaccccag gct 53
<210> 8
   <211> 20
   <212> DNA
   <213> artificial sequence
<220>
   <223> primer
<400> 8
   acccagaaga ctgtggatgg 20
<210> 9
   <211> 20
   <212> DNA
   <213> artificial sequence
<220>
   <223> primer
<400> 9
   ggagacaacc tggtcctcag 20
<210> 10
   <211> 30
   <212> DNA
   <213> artificial sequence
<220>
   <223> primer
<400> 10
   ccatcgatta atggctcgtc gcgcgcgggc 30
<210> 11
   <211> 30
   <212> DNA
   <213> artificial sequence
<220>
   <223> primer
<400> 11
   ggatcgatga tccatggggt caaactgcat 30
<210> 12
   <211> 30
   <212> DNA
   <213> artificial sequence
<220>
   <223> primer
<400> 12
   ccatcgatta atggagacag acacactcct 30
<210> 13
   <211> 32
   <212> DNA
   <213> artificial sequence
<220>
   <223> primer
<400> 13
   agttaaaacc gtacgcgtca ccagtggaag gt 32
<210> 14
   <211> 63
   <212> DNA
   <213> artificial sequence
<220>
   <223> primer
<400> 14
   ggggacaagt ttgtacaaaa aagcaggctt cttctgtacc aagagccgac gtgctgagcg 60
   tta 63
<210> 15
   <211> 70
   <212> DNA
   <213> artificial sequence
<220>
   <223> primer
<400> 15
   ggggaccact ttgtacaaga aagctgggtc ggcctcactg tagcactcgt agatgttgtc 60
   ctccatcaaa 70
<210> 16
   <211> 36
   <212> DNA
   <213> artificial sequence
<220>
   <223> primer
<400> 16
   ggacctcatt aacgcctcgg ccgccaagaa ccccaa 36
<210> 17
   <211> 38
   <212> DNA
   <213> artificial sequence
<220>
   <223> primer
<400> 17
   cggccgaggc gttaatgagg tccaccagca gctccttc 38
<210> 18
   <211> 35
   <212> DNA
   <213> artificial sequence
<220>
   <223> primer
<400> 18
   ggacctggac aacacctcag tggtggaaga cggcc 35
<210> 19
   <211> 38
   <212> DNA
   <213> artificial sequence
<220>
   <223> primer
<400> 19
   ccactgaggt gttgtccagg tcccgaagga tgtagctc 38
<210> 20
   <211> 55
   <212> DNA
   <213> artificial sequence
<220>
   <223> primer
<400> 20
   ggggacaagt ttgtacaaaa aagcaggctt cgcctcggcc gccaagaacc ccaag 55
<210> 21
   <211> 57
   <212> DNA
   <213> artificial sequence
<220>
   <223> primer
<400> 21
   ggggaccact ttgtacaaga aagctgggtc ctagtcgtcc aggtcccgaa ggatgta 57
<210> 22
   <211> 43
   <212> DNA
   <213> artificial sequence
<220>
   <223> primer
<400> 22
   catgctggcc gccacagagt ctgtggtgga gaagatgctc acc 43
<210> 23
   <211> 40
   <212> DNA
   <213> artificial sequence
<220>
   <223> primer
<400> 23
   ctctgtggcg gccagcatga gcttggggtt cttggcggcc 40
<210> 24
   <211> 37
   <212> DNA
   <213> artificial sequence
<220>
   <223> primer
<400> 24
   cctctcgcct tctgggtgaa catcctgaac ccccagt 37
<210> 25
   <211> 40
   <212> DNA
   <213> artificial sequence
<220>
   <223> primer
<400> 25
   ccagaaggcg agaggaaggc tgttggtctt ccagatgtgt 40
<210> 26
   <211> 58
   <212> DNA
   <213> artificial sequence
<220>
   <223> primer
<400> 26
   ccgggctgct gaaagtaatc acaatctcga gattgtgatt actttcagca gctttttg 58
<210> 27
   <211> 58
   <212> DNA
   <213> artificial sequence
<220>
   <223> primer
<400> 27
   ccggccttgt ctattccctg aacttctcga gaagttcagg gaatagacaa ggtttttg 58

## Description

The present invention is related to the treatment of certain cancers using antagonists of the binding between a Plexin and a semaphorin.

Plexins are a family of transmembrane receptors consisting of nine members in vertebrates, divided into four subfamilies: PlexinA(1-4), PlexinB(1-3), PlexinC1 and PlexinD1. Plexins are part of the semaphorin gene superfamily, which also includes the semaphorin ligands and the receptor tyrosine kinases Met and RON. All of these proteins are characterized by an N-terminal 500-amino-acid sema domain that is essential for protein function. The structure of the sema domain is a seven-blade β-propeller fold that has overall structural similarity to the extracellular domain of α-integrins (Gherardi et al., 2004; Koppel et al., 1997). Next to the sema domain, plexins contain two to three repeats of the plexin-semaphorin-integrin (PSI) domain, a cysteine-rich motif also referred as a MET-related sequence (MRS) (Bork et al., 1999), and three IPT (Ig-like fold shared by plexins and transcription factors) domains. The cytoplasmic domain of plexins is highly conserved between all members of the family and contains homology to GTPase-activating proteins (GAPs), which catalyze the inactivation of R-Ras GTPase (Rohm et al., 2000a,b; Takahashi et al., 1999; Tamagnone et al., 1999; Oinuma et al., 2004). Plexins are further characterized by specific protein domains such as cleavage sites for furin-like pro-protein convertases in the extracellular domains of PlexinB1 and PlexinB2, and binding sites for PDZ (PSD-95/Dlg/ZO-1) domains in the C-terminus of PlexinD1 and B-type plexins. PlexinD1 is the most divergent plexin family member that includes an atypical feature in its extracellular domain: the third IPT motif in PlexinD1 contains only six of the eight conserved cysteins normally encountered in a IPT domain (Van der Zwaag et al., 2002).

Plexins are the main signaling receptors for Semaphorins family of ligands, which consist of more than 20 proteins (in vertebrates), divided into different classes on the basis of sequence similarity and structural organisation (Semaphorin Nomenclature Committee, 1999). Semaphorins were initially identified as axonal guidance cues in the developing nervous system. However, it is now clear that in addition to their central role in the nervous system, Semaphorins are implicated in a range of processes, including vascular patterning, tissue morphogenesis, tumor formation, and play important roles in the mature immune system (Mann et al., 2007; Carrapucia and Tamagnone, 2009).

Plexins function as high affinity receptors for membrane-bound semaphorins (class 4-7). However, most secreted semaphorins (class 3) do not appear to bind plexin receptors directly. Instead, they bind to receptor complexes that include members of the Neuropilin family as ligand-binding subunit and a Plexin signal-transducing element. One exception to this rule is the secreted Semaphorin 3E (Sema3E) which binds with high affinity to PlexinD1 receptor, independently of the presence or absence of Neuropilins (Gu et al., 2005).

PlexinD1 is prominently expressed in most, if not all, endothelial vascular cells during embryogenesis (Van der Zwaag et al., 2002; Gitler et al., 2004). Expression in other organs has been demonstrated, including central and peripheral nervous system (van der Zwaag et al., 2002; Chauvet et al., 2007; Pecho-Vrieseling et al., 2009), salivary gland (Chung et al., 2007), neural crest cells (Toyofuku et al., 2008), osteoblasts (Kanda et al., 2007) and lymphocytes (Choi et al., 2008).

Cardiovascular development is notably abnormal in mice with a targeted inactivation of *PlexinD1.* Structural defects involve the outflow tract of the heart and derivatives of the aortic arch arteries, coronary and peripheral blood vessels (Gitler et al., 2004). Inactivation of PlexinD1 also leads to axial skeletal morphogenesis defects (Kada et al., 2007) that appear to be secondary to vascular patterning defects (Zhang et al., 2008). In addition to endothelial cell defects, disruption of *PlexinD1* function causes abnormal growth and guidance of neuronal axons in the nervous system (Chauvet et al., 2007; Pecho-Vrieseling et al., 2009).

The primary ligand for PlexinD1 is Sema3E (Gu et al., 2005). Sema3E/PlexinD1 interaction is not only functionally important in the vascular system of mouse embryos to pattern intersomitic blood vessels (Gu et al., 2005) but also in the nervous system for neural circuit assembly (Chauvet et al., 2007; Pecho-Vrieseling et al., 2009). However, PlexinD1 may also participate in receptor complexes for other semaphorin ligands. This is suggested by the more severe perinatal lethality of the *PlexinD1*^{*-*/*-*} mutation as compared to *Sema3E*^{*-*/*-*} mice (Gu et al., 2005), and by the ability of complexes of PlexinD1 with Npn-1 or Npn-2 to bind Sema3A, Sema3C and/or Sema4A *in vitro* (Gitler et al., 2004; Toyofuku et al., 2007).

Sema3E acts on PlexinD1 to inhibit migration and capillary tube formation of endothelial cells, by inducing detachment and retraction of the cells from the extracellular matrix (Gu et al., 2005; Toyofuku et al., 2007; Sakurai et al., 2010). At the molecular level, this process involves the association of PlexinD1 with R-Ras and concomitant activation of Arf6, which affects the activation status of integrins and their intracellular trafficking (Sakurai et al., 2010). Sema3E/PlexinD1 may also inhibit endothelial cell growth and tube formation by suppressing the vascular endothelial growth factor (VEGF) signaling pathway (Moriya et al. 2010). Whereas Sema3E has emerged as a potent anti-angiogenic factor, another study has reported that a furin-cleaved form of Sema3E exert a motogenic effect on endothelial cells (Christensen et al., 2005). The underlying molecular mechanism is not known. In the nervous system, the dual activity of Sema3E involves different receptor complexes. Sema3E acts on PlexinD1 to inhibit and repel neuronal axon growth (Chauvet et al., 2007). This requires stimulation of PlexinD1 R-Ras GAP activity by Rnd2 GTPases, inhibition of R-Ras function and subsequent inactivation of phosphatidylinositol-3 kinase (PI3K)/Akt and activation GSK-3β (Uesugi et al., 2009; Bellon et al., 2010). On the other hand, Sema3E attracts and stimulates axon growth by acting through a trimeric receptor complex that comprises, in addition to PlexinD1, Neuropilin-1 and the vascular endothelial growth factor receptor 2 (VEGFR2) co-receptors (Chauvet et al., 2007; Bellon et al., 2010). In the later case, PlexinD1 serves as a ligand-binding subunit for Sema3E ligand but does not transduce intracellular signaling. Instead, Sema3E triggers VEGFR2-dependent activation of the PI3K/Akt pathway that is required for the increase in axonal growth (Bellon et al., 2010).

Plexin and Neuropilin receptor expression have been reported in a number of human cancers. PlexinD1 expression is found on tumor-associated blood vessels and on tumor cells in a large variety of clinical tumors of both primary and metastatic origins. These include breast carcinoma, renal cell carcinoma, prostate adenocarcinoma, gastric adenocarcinoma, glioblastoma, medulloblastoma, melanoma, as well as brain metastasis of adenocarcinoma, glioblastoma, neuro-endocrine lung tumor and melanoma (Roodink et al., 2009). PlexinD1 expression has also been reported in tumor-derived cells from breast origin (Kigel et al., 2008). A positive correlation between PlexinD1 expression and malignancy grade has been reported in a human melanoma progression series: PlexinD1 is absent in benign melanocytic lesions, whereas expression is abundant in both invasive primary and disseminated melanoma (Roodink et al., 2008). This is suggestive of a role of PlexinD1 in cancer progression. So far, however, no studies have directly addressed the biological function of PlexinD1 in cancer.

Several studies have shown the importance of Semaphorin signalling in cancer progression, through either anti-tumorigenic or tumorigenic function. Sema3E has been initially isolated from mouse mammary adenocarcinoma cell lines (Christensen et al., 1998). Sema3E expression has been observed in human breast and prostate cancer cell lines (Christensen et al., 2005; Herman et al., 2004; Kigel et al., 2008), human breast cancer and lymph node metastasis (Christensen et al., 2005), and human melanoma (Roodink et al., 2008). Sema3E has been proposed to act as a mediator of tumor invasion and metastasis. Indeed, a positive correlation exists between Sema3E expression in mouse adenocarcinoma cell lines and the ability of the cells to form metastasis in animal tumor models (Christensen et al., 1998). Furthermore, forced expression of Sema3E in non-metastatic cells induces the ability to form experimental lung metastasis (Christensen et al., 2005). Christensen 2005 is using 168FARN cells which are mammary tumor cells. When the cells are injected/grafted in animal, overexpression of Sema3E has no effect on the growth of the primary tumor but stimulates formation of pulmonary tumors (metastasis). Thus Christensen indicates that Sema3E is not required for primary tumor growth. On the other hand, Sema3E shows reduced expression in invasive melanomas and disseminated metastasis, compared to benign melanocytic lesions (Roodink et al., 2008), suggesting an anti-tumorigenic function. In support of this idea, expression of Sema3E in cancer cells inhibits *in vivo* tumor development in animal models of breast cancer and melanoma (Roodink et al., 2008; Kigel et al., 2008) and reduces metastatic potential of melanoma xenograph (Roodink et al., 2008). Therefore, Sema3E signaling may have distinct and opposite effects.

The mechanisms by which Sema3E regulates tumor progression remain unclear. Tumor cells may secrete Sema3E to regulate tumor-cell behaviour and/or to act on different cell types in the tumor environment. When overexpressed in cancer cell lines, Sema3E functions in an autocrine manner to decrease invasion and adhesion of prostate cancer cells *in vitro* (Herman and Meadows, 2007) and diminish colony formation of breast cancer cells in soft agar assay (Kigel et al., 2008). Several studies also reported that Sema3E overexpressed by melanoma and breast cancer-derived cells acts on endothelial cells, resulting in decreased tumor angiogenesis *in vivo* (Roodink et al., 2008; Kigel et al., 2008). Whether these effects are mediated by PlexinD1 receptor remains to be determined.

WO01/14420 discloses nucleic acid sequences encoding several Plexins, such as the Plexins B2, B3, D1 and A4 that are defined as novel Plexins at this time. Antibodies, molecules binding to the Plexins and chimeric molecules, including a Plexin extracellular domain fused to an immunoglobulin are described. The applications are essentially the modulation of cell growth (i.e. nerve growth) and immune regulation. Extension is made without any basis on the treatment of cancer in general. Also, the basis of this previous work is the signaling pathway between a Plexin and a Neuropilin, and there is no experiment on PlexinD1. Roodink et al. (Cancer Research, American Association for cancer research, 2005, vol.65, 18:8317-8323) discloses that PlexinD1 is expressed by vascular cells associated with tumors (angiogenesis) and some cancer cells as well. However if the functional role of PlexinD1 in developmental angiogenesis is now well established, whether PlexinD1 on tumor cells is functionally important is not known.

WO2007/009816 is related to the implication of PlexinD1 in angiogenesis and proposed that PlexinD1 could be a targetable protein in the treatment of angiogenesis, using molecules that interfere in the interaction between PlexinD1 and its ligands, interfere in the expression of the PlexinD1 gene or that capture PlexinD1 ligands. Ligands of PlexinD1 are mentioned to include neuropilin-1, neuropilin-2, semaphorin 3C, semaphorin 3E, VEGF-receptor 1, VEGF-receptor 2, VEGF-A. In particular, this document proposes binding molecules which are able to bind PlexinD1 and inhibit the ligand-induced GTPase signalling by PlexinD1 or the migration of cells, these mechanisms being involved in angiogenesis. A recombinant protein is proposed, that consists of the extracellular domain of PlexinD1 fused to a constant region of a human heavy chain.

Also, US 7,482,322 is related to the PlexinD1 derived polypeptides for use in the inhibition of angiogenesis.

The literature completely fails to teach or demonstrate that blocking the direct PlexinD1/Sema3E binding could be of help in the treatment of certain cancers. The fact that Sema3E signaling may have distinct and opposite effects may explain this failure.

The present inventors focused their research on the determination of how blocking endogenous Sema3E/PlexinD1 interaction affects tumor cell behaviour and cancer progression. The objective of the present invention was indeed to propose new therapeutics approaches for the treatment of cancer, in particular aggressive tumours and metastases.

The present inventors have demonstrated that a peptide called SD1, comprising the N-terminal sema domain of approximately 500 amino acids from PlexinD1 specifically, binds to Sema3E and antagonizes the Sema3E/PlexinD1 interaction. The inventors have further demonstrated that this binding inhibits the Sema3E/PlexinD1 signaling. In further experiments, they have demonstrated *in vivo* that SD1 inhibits the development of primary tumors and metastasis and induces tumor cell death. They have then demonstrated that blocking Sema3E autocrine activity induces PlexinD1-mediated death of tumor cells, that PlexinD1 is a dependence receptor and has a pro-apoptotic function. The inventors have thus demonstrated that antagonising the Sema3E/PlexinD1 interaction may be beneficial for a patient having a cancer with Sema3E expression and that the sema domain of PlexinD1 is a valuable candidate as antagonist or as part of an antagonist.

A first object of the invention is thus an antagonist as defined in the claims. "With Sema3E expression" means autocrine expression by tumoral cells and/or paracrine expression by the stroma or other surrounding cells.

In a preferred embodiment, the cancer is one expressing Sema3E at a level higher than a non-tumoral tissue of the same nature or at a level higher than a tumoral tissue of the same nature and namely of lower tumoral grade. In particular, the comparison may be realized with the mean level of Sema3E expression in a panel of tissues of the same nature, or realized with the mean level of biopsies of tumors of the same nature and namely of lower tumoral grade.

The application discloses that the cancer is a metastatic breast cancer with Sema3E expression.

In an embodiment, the cancer is a lung cancer with Sema3E expression.

This list is not limitative, as it is possible for the person skilled in the art to test Sema3E expression in any cancer that the practitioner is faced to.

Therefore, in other embodiments, the cancer is for example selected from the group consisting of those listed thereafter : breast cancer, prostate cancer, melanoma, glioblastoma, with Sema3E expression.

In an embodiment, breast cancer is one with axilary node involvement.

In another embodiment, breast cancer is one with distant metastases.

In an embodiment, the use is for the inhibition or the cease of primary cancer cell development, wherein the tumor cells and/or the stroma express Sema3E.

In another embodiment, the use is for the further inhibition or the cease of metastasis development, wherein the tumor cells and/or the stroma express Sema3E.

In still another embodiment, the use is for inducing PlexinD1-mediated death of tumor cells or tumor cell apoptosis, wherein the tumor cells and/or the stroma express Sema3E.

The application also discloses the use of such an antagonist according to the invention, as describes above and below, for the manufacture of a drug or medicament for treating :
- a cancer with Sema3E expression,
- primary cancer cell development, with Sema3E expression,
- metastasis development, with Sema3E expression, and/or
- PlexinD1-mediated death of tumor cells or tumor cell apoptosis, wherein Sema3E expression is present.

The term "antagonist" is used in the broadest sense, and includes any molecule that specifically binds to PlexinD1 or Sema3E and blocks the Sema3E/PlexinD1 binding. This encompasses: a molecule that binds to the PlexinD1's Sema3E-binding region, typically a molecule that bind to the sema domain of PlexinD1; a molecule that binds to the Sema3E's PlexinD1-binding region, typically a molecule that bind to the Sema3E's PlexinD1 sema domain-binding region.

In an embodiment, the antagonist is a polypeptide. This polypeptide is a polypeptide comprising or consisting of a PlexinD1 domain binding to Sema3E or a Sema3E domain binding to PlexinD1.

In a preferred embodiment, the polypeptide comprises or consists of a PlexinD1 sema domain, which includes a fragment of the sema domain which keeps the function of the whole domain, say it is able to specifically binds to Sema3E and blocks the Sema3E/PlexinD1 binding. Preferably, the polypeptide comprises the sema domain. As it will appear from the present description, the sema domain may be combined with additional sequences such as a secretion signal sequence and/or tags such as myc tags for process conveniency.

The "polypeptide" includes both a native sequence sema domain, a sema domain variant, and a chimeric sema domain.

"Native sema domain" comprises a sema polypeptide having the same amino acid sequence as the corresponding polypeptide found in the human or derived therefrom. The native sema polypeptide can be natural, i.e. isolated from human, recombinant, i.e. produced by recombinant means, or synthetic, i.e. produced by synthesis.

The native sema domain encompasses the full-length amino acid sequence of the corresponding sema domain found in the human or a naturally-occurring truncated or secreted form. It also encompasses a fragment of the full-length amino acid sequence which is capable of binding Sema3E. It encompasses the whole extracellular domain of PlexinD1.

In an embodiment, the sema domain comprises or consists of the amino acid sequence SEQ ID NO:1.

In another embodiment, the sema domain comprises or consists of the amino acid sequence encoded by the nucleotide sequence SEQ ID NO:2.

"sema domain variant" means a polypeptide which amino acid sequence differs from the corresponding native sequence of the sema domain and keep the function of the native sema domain. Such a variant or a fragment may have at least about 80, 81, 82, 83, 84, 85, 86, 87, 88, 89, 90, 91, 92, 93, 94, 95, 96, 97, 98 or 99% amino acid sequence identity with the corresponding, either full-length or partial (fragment), native sema domain polypeptide, such as the sequence depicted on SEQ ID NO:1.

"Chimeric sema domain" encompasses a sema domain (including fragment and variant) fused to a heterologous amino acid sequence.

In an embodiment, the chimeric sema polypeptide is a fusion protein comprising a sema domain polypeptide and an immunoglobin domain. "Immunoglobin domain" means a Fc domain, a heavy chain or a light chain.

In a preferred embodiment, the immunoglobin domain is a Fc sequence. It may be in particular a Fc from a human IgG1.

In an embodiment, the IgG Fc fragment comprises or is made of the amino acid sequence SEQ ID NO:3.

In a preferred embodiment, the antagonist is made of or comprise a fusion protein comprising SEQ ID NO:1 and SEQ ID NO:3.

"Fragment" means at least 5, preferably at least 8, more particularly at least 10, 15, 20, 30, 40, 50 or 100 continuous amino acids sequence of the native amino acid sequence or of one of its variants according to the invention.

In another embodiment, the polypeptide comprises or consists of a Sema3E amino acid sequence, which is the binding region for PlexinD1. This includes a fragment of the binding region which keeps the function of the whole region, say it is able to specifically binds to PlexinD1 and blocks the Sema3E/PlexinD1 binding. As for the sema domain, the polypeptide may be native, variant or chimeric.

In another embodiment, the antagonist is an antibody which is able to bind to either PlexinD1 or Sema3E in a manner that blocks Sema3E/PlexinD1 binding. The antibody may be specific for the Sema3E/PlexinD1 binding sequence.

In an embodiment, the antagonist is an antibody against PlexinD1 sema domain.

In still another embodiment, the antagonist is an antibody against the sema binding sequence of Sema3E.

"Antibody" is used in the broadest sense to designate any antibody that may bind to PlexinD1 or Sema3E wherein this binding impedes the binding between Sema3E and PlexinD1.

"Antibody" includes monoclonal antibodies, polyclonal antibodies, single-chain antibodies and antigen binding fragments of these antibodies which exhibit the desired biological activity. The monoclonal antibodies may be murine, chimeric or humanized. The term "antibody" refers to any full-length antibody or functional fragment of an antibody (obtained by genetic engineering or not), comprising, or consisting of, at least one antigenic combination site, allowing said antibody to bind to at least one antigenic determinant of an antigenic compound. By way of example of antibody fragments, there may be mentioned the fragments Fab, Fab', F(ab')₂ and the single-chain variable fragments (scFv chains). The antibodies used in the present invention are antibodies specific for the antigen. They are preferably monoclonal antibodies or monospecific polyclonal antibodies, that is to say that they specifically recognize only one epitope. The production of monoclonal antibodies or of monospecific polyclonal sera, or of antibodies obtained by screening genomic libraries, useful in the context of the invention are conventional techniques.

An anti-PlexinD1 or anti-Sema3E polyclonal antibody may, inter alia, be obtained by immunizing an animal such as a rabbit, a mouse and the like with the aid of the selected amino acid sequence, collecting and then depleting the antiserum obtained on, for example, an immunoadsorbent containing the receptor according to methods known per se to a person skilled in the art.

The native sema domain amino acid sequence is as depicted on SEQ ID NO:1 and may be used in whole or in part to design antibodies. Variant sequences may also be used as described below.

The native amino acid sequence of the binding region of Sema3E may be used in whole or in part to design antibodies. Variant sequences may also be used as described below.

Generally, other monoclonal antibodies may be obtained according to the conventional method of lymphocyte fusion and hybridoma culture described by Köhler and Milstein, (1975). Other methods for preparing monoclonal antibodies are also known (Harlow et al., ed., 1988 "Antibodies: a laboratory manual"). The monoclonal antibodies may be prepared by immunizing a mammal (for example a mouse, a rat, a rabbit or even a human being, and the like) and using the lymphocyte fusion technique leading to hybridoma (Köhler and Milstein, 1975).

Alternative techniques to this customary technique exist. It is possible, for example, to produce monoclonal antibodies by expressing a nucleic acid cloned from a hybridoma. It is also possible to produce antibodies by the phage display technique by introducing cDNAs for antibodies into vectors, which are typically filamentous phages which exhibit gene libraries V at the surface of the phage (for example fUSE5 for *E. coli,* Scott, 1990). Protocols for constructing these antibody libraries are described in Marks et al. (1991).

The application also discloses that the agonist is a siRNA which is capable of inhibiting Sema3E expression (silencing).

A small interfering RNA or siRNA is a double stranded RNA (dsRNA) that may have from 10 to 50 nucleotides in length and which reduces expression of the target gene. Portions of the first strand are complementary to the target gene, i.e. it has sufficient complementarity to hybridize to the target gene, for example there is at least 80% identity to the target gene or to a portion thereof.

For their use in human treatment, the antagonists or active principles according to the invention may be included in a pharmaceutical composition that also contains a pharmaceutically acceptable excipient or vehicle. These compositions are thus intended for the uses that have been described above.

The application also discloses a pharmaceutical composition or a kit of parts, containing an antagonist according to the invention and a pharmaceutically acceptable excipient or vehicle.

The application also discloses a therapeutic treatment comprising administering a sufficient amount of an antagonist according to the invention, or a pharmaceutical composition containing this antagonist as an active principle, to a patient in need thereof.

A patient in need thereof is a patient having tumor cells that express Sema3E in accordance with the invention.

According to one feature, the therapeutic treatment aims at inducing:
- cancer regression,
- inhibition of primary cancer cell development,
- inhibition of metastasis development, and/or
- PlexinD1-mediated death of tumor cells or tumor cell apoptosis.

By "effective amount" is meant an amount sufficient to achieve a concentration of antagonist which is capable of blocking Sema3E/PlexinD1 binding and inducing the therapeutic effect so as to prevent or treat the disease to be treated. Such concentrations can be routinely determined by those of skilled in the art. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, etc. It will also be appreciated by those of stalled in the art that the dosage may be dependent on the stability of the administered peptide.

The treating a cancer is meant a method aiming at curing, improving the condition and/or extending the lifespan of an individual suffering from a cancer according to the invention.

As the method intends to treat those patients having a tumour with Sema3E expression, especially high Sema3E expression, the method of treatment may comprise a previous step intended to check whether or not such Sema3E expression is present in the patient and the treatment with the antagonist is done only on patients that respond positive.

The application also discloses a method of therapeutic treatment comprising at least two phases, wherein the first phase consists in determining whether or not the patient has a tumour with Sema3E expression and the second phase consists in the administration of a sufficient amount of an antagonist according to the invention, or a pharmaceutical composition containing this antagonist as an active principle, to a patient responding positively to the first phase.

The application also discloses the use of an antagonist according to the invention, or a pharmaceutical composition containing this antagonist, as an active principle for treating a patient in need thereof, with such a patient being of the type that has just been defined.

In another aspect, the present invention is directed to an *in vitro* method for selecting a compound for the prevention or the treatment of a cancer with Sema3E expression and wherein the cancer cells bear PlexinD1, wherein said method comprises the following steps of :
a) having a medium containing Sema3E, or a fragment thereof, and PlexinD1 receptor, or a fragment thereof, wherein said Sema3E, or a fragment thereof, and said PlexinD1 receptor, or a fragment thereof, is able to specifically interact together to form a binding pair;
b) contacting said medium with the compound to be tested ;
c) measuring the inhibition of the interaction between Sema3E, or a fragment thereof, and said PlexinD1 receptor, or a fragment thereof.

Compounds that inhibits the interaction are selected. This selection may be performed if the measuring in step c) demonstrates a significant inhibition of said interaction.

The inhibition of this interaction can be obtained for example by the complete or partial inhibition of the binding of Sema3E to its receptor. In an embodiment, this is performed in presence of a competitive ligand (such as an antibody which is directed to this extracellular membrane domain of said receptor), or in presence of a compound able to form a specific complex with Sema3E (such as a molecule comprising the sema domain of PlexinD1).

In an preferred embodiment, the method according to the present invention is characterized in that at step a) :
- said Sema3E comprises or is the Sema3E binding region which is able to interact with PlexinD1 ; and/or
- said PlexinD1 receptor fragment comprises or is the PlexinD1 binding region which is interact with Sema3E.

In another embodiment, the method according to the present invention is characterized in that the PlexinD1 binding domain is the sema domain.

In another preferred embodiment, the method according to the present invention is characterized in that at step c) the measure of the inhibition of the interaction is carried out by immunoassay (particularly by ELISA or by Immunoradiometric Assay (IRMA)), by Scintillation Proximity Assay (SPA) or by Fluorescence Resonance Energy Transfer (FRET)

In another particular preferred embodiment, the method according to the present invention is characterized in that at step a) said medium contains cells which express at their surface membrane an endogenous or a recombinant PlexinD1 receptor or a fragment thereof, particularly a recombinant sema domain.

In another particular preferred embodiment, the method according to the present invention is characterized in that at step a) said medium contains tumoral cells, preferably metastatic tumoral cells, which express endogenously said PlexinD1 receptor at their membrane surface or a fragment thereof, particularly a recombinant sema domain and which expresses or overexpresses Sema3E, or a fragment thereof and wherein at step c) the inhibition of the interaction between Sema3E and its receptor in presence of the compound to be tested, is measured by the apoptosis of cells death induced by the presence of the compound to be tested, preferably analysed using the trypan blue staining method. It may be tumoral cells from metastatic tumoral cell line, preferably 4T1 cells. Other cell may be used such as those used in the examples: MDA-MB-231, MCF-7.

"Cell death" can readily be determined by one skilled in the art. For instance, the staining of cells with vital dyes is a commonly used approach to quantify cell death.

The application also discloses a kit for the selection of a compound for the prevention or the treatment of cancer with Sema3E expression, wherein said kit comprises :
- PlexinD1 receptor protein, or a fragment thereof able to specifically interact with the Sema3E protein to form a binding pair, preferably recombinant protein, preferably sema domain or protein comprising the sema domain ; and
- Sema3E protein, or a fragment thereof able to specifically interact with said receptor protein to form a binding pair, preferably recombinant protein.

In a preferred embodiment, said kit comprises tumoral cells which express PlexinD1 receptor and which express or overexpress Sema3E, particularly cells from metastatic tumoral cell line, preferably 4T1 cells. Other cell may be used such as those used in the examples: MDA-MB-231, MCF-7

The kit can comprise a labeled compound or agent capable of quantifying these proteins or the binding events.

In still another aspect, the present invention is directed to an *in vitro* method for predicting the presence of a cancer with Sema3E expression, and optionally of PlexinD1, in a patient from a biological sample (biopsy or blood or serum sample, and the like, comprising tumoral cells) of said patient containing tumors cells, said method comprising the step of measuring of the Sema3E expression level in said biological sample and optionally of PlexinD1. In an embodiment, this method comprises the step of selecting the patient expressing Sema3E as suitable to be treated with an antagonist according to the invention, which inhibits the interaction between PlexinD1 and Sema3E. This method may be carried out just after the cancer is diagnosed, or during anti-cancer treatment to follow-up evolution of the disease, i.e. to test for emergence, or on the contrary, for disappearance of tumoral cells expressing Sma3E.

The application further discloses kits that are useful in the above method. Such kits comprise means for detecting the amount and/or expression level of Sema3E and optionally means for detecting the amount and/or expression level of PlexinD1. This kit may be used for prognosing the outcome of a cancer in a patient, for designing a treatment regimen, for monitoring the progression of the cancer, and/or for monitoring the response of the individual to a drug (i.e. "drug monitoring").

Means for detecting the amount and/or expression level of Sema3E are well-known in the art. They include, e.g. reagents allowing the detection of Sema3E mRNA by real-time quantitative-PCR, such as primers specific for Sema3E. When the kit comprises means for real-time quantitative-PCR Sema3E mRNA detection, the kit may further comprise a second reagent, labeled with a detectable compound, which binds to Sema3E mRNA synthesized during the PCR.

Means for detecting the amount and/or expression level of Sema3E may also include antibodies specifically binding to Sema3E. Such means can be labeled with detectable compound such as fluorophores or radioactive compounds. For example, the probe or the antibody specifically binding to Sema3E may be labeled with a detectable compound. Alternatively, when the kit comprises an antibody, the kit may further comprise a secondary antibody, labeled with a detectable compound, which binds to an unlabelled antibody specifically binding to Sema3E.

The means for detecting the amount and/or expression level of Sema3E may also include reagents such as e.g. reaction, hybridization and/or washing buffers. The means may be present, e.g., in vials or microtiter plates, or be attached to a solid support such as a microarray as can be the case for primers and probes.

In certain embodiments of the methods or kits of the present invention, the determination of the transcripts involves the use of a probe/primer in a polymerase chain reaction (PCR), such as anchor PCR or RACE PCR, or, alternatively, in a ligation chain reaction (LCR) (see, e.g. Landegran et al., 1988, Science 241:23-1080 ; and Nakazawa et al., 1994, Proc. Natl. Acad. Sci. USA, 91:360-364), or alternatively quantitative real time RT-PCR. This method can include the steps of collecting a sample of cells from a patient, isolating nucleic acid (e.g. mRNA) from the cells of the sample, optionally transforming mRNA into corresponding cDNA, contacting the nucleic acid sample with one or more primers which specifically hybridize to the Sema3E or mRNA or their corresponding cDNA under condition such that hybridization and amplification of the Sema3E mRNA or CDNA occurs, and quantifying the presence of the amplification products. It is anticipated that PCR and/or LCR may be desirable to uses as an amplification step in conjunction with any of the techniques used for quantifying nucleic acid detection.

The methods described herein may be performed, for example, by utilizing prepackaged diagnostic kits comprising at least one probe nucleid acid or set of primer or antibody reagent described herein, which may be conveniently used, e.g. in clinical settings to follow-up or diagnose patients.

The present invention will now be described in more details using non-limiting examples, which refer to the following figures.

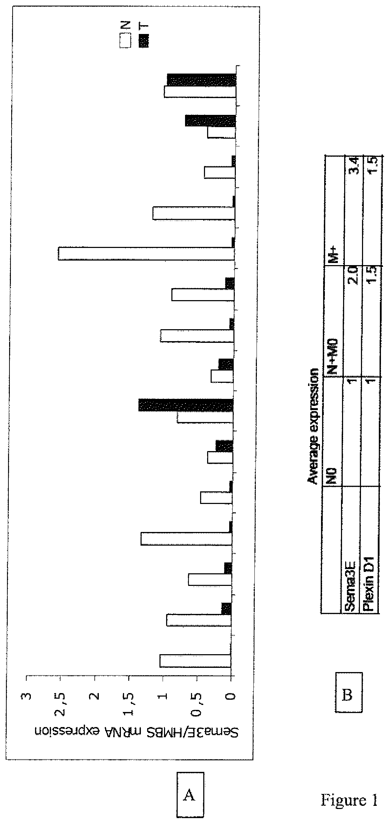

**Figure 1****. *PlxnD1* and *Sema3E* expression in human cancers**
   (A) Sema3E up-regulation detected in non small cell lung cancer. Expression profile of Sema3E examined by quantitative real-time PCR using total RNA extracted from 15 tumor biopsies couples obtained from patients with non small cell lung cancer tumors (T). HPRT was used as the reference gene because of its weak variability between normal and tumoral tissue. The amount of Sema3E is compared between Tumoral tissue (T) and adjacent normal tissue (N). Over-expression of Sema3E is observed for 2 patients among 15 (13%).
   (B) Sema3E up-regulation detected in breast cancer. Expression profile of Sema3E and PlexinD1 was examined with quantitative real-time RT-PCR (detailed results not shown). RT-PCR was performed by using total RNA extracted from 67 tumor biopsies. They were obtained from patients with tumors localized to the breast (N0), with only axillary node involvement (N+M0), and with distant metastases at diagnosis (M+). Specific human Sema3E primers and primers corresponding to the human HMBS gene (hydroxymethylbilane synthase) were used. HMBS was used as a reference here because it only shows a weak variability at the mRNA level between normal and breast tumoral tissues, as described (de Kok et al. Lab Invest 2005 Jan; 85(1):154-9). Another reference TBP was also used with similar results (data not shown). Sema3E is given as the ratio between Sema3E expression in each sample and the average of Sema3E expression in the N0 samples. PlexinD1 expression is also given as the ratio between PlexinD1 expression in each sample and the average of PlexinD1 in N0 samples. Student test was used; the significant p value for Sema3E is p = 0.0369. The average of Sema3E and PlexinD1 expression is indicated for each tumor stage.

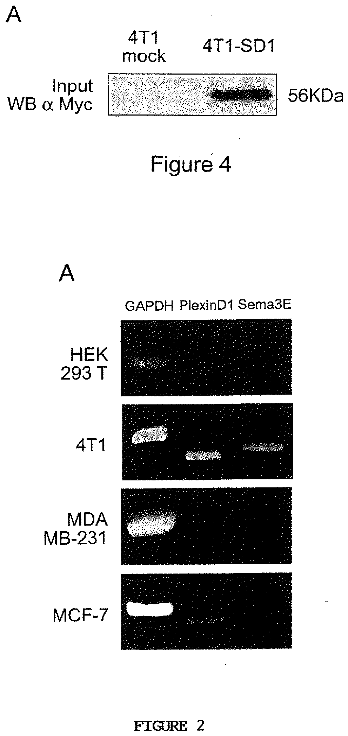

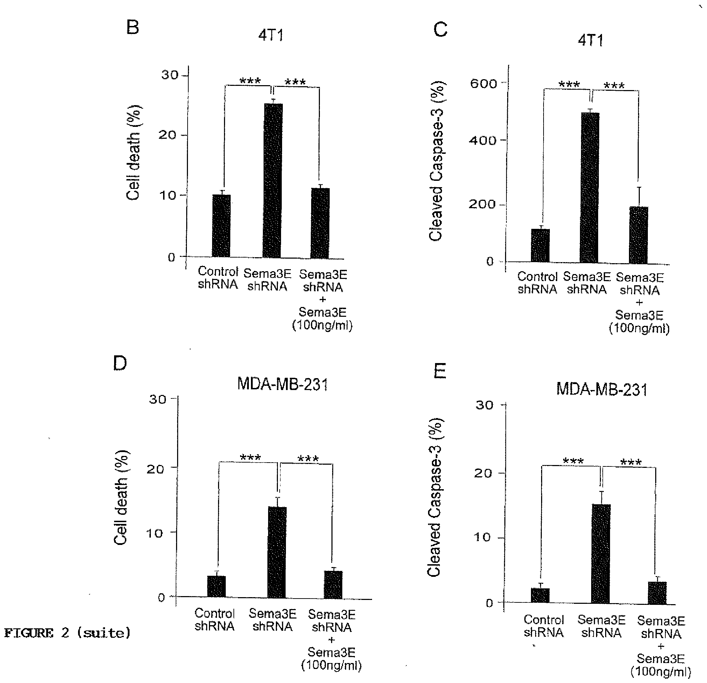

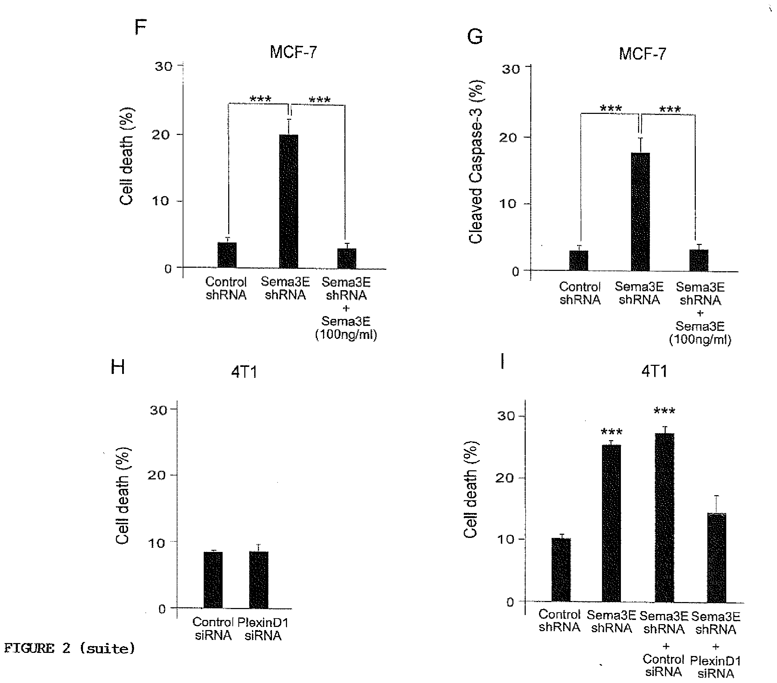

**Figure 2****. Induction of cell death by inhibiting Sema3E in aggressive mouse and human cancer cell lines expressing Sema3E and PlexinD1, is mediated by PlexinD1.**
   (A) RT-PCR analysis of *PlexinD1* and *Sema3E* mRNAs expression in 4T1, MDA-MB-231 and MCF-7 cell lines. *GAPDH* mRNA was used as an internal control and HEK 293T cells as negative control. (B-G) Survival effect of Sema3E in cancer cells. 4T1 (B-C), MDA-MB-231 (D-E) and MCF-7 (F-G) cells were transfected with control shRNA or shRNA specific to mouse Sema3E for 4T1 and human Sema3E for MDA-MB-231 and MCF-7 cells, in the absence or presence of exogenous Sema3E (100ng/ml). Cell death was evaluated by trypan blue-exclusion assay (B, D, F) or by quantification of the percentage of cleaved Caspase-3 positive cells (C, E, G). Exogenous Sema3E rescues 4T1, MDA-MB-231 and MCF-7 cells from cell-death induced by knockdown of Sema3E. (H-I) PlexinD1 inhibition by mouse siRNA to PlexinD1 does not affect 4T1 cell death evaluated by trypan blue exclusion (H) but abolishes Sema3E shRNA induced cell death (I). Data are presented as average ± SEM (n = 3) *significantly different with p< 0.05. **significantly different with p< 0.01. ***significantly different with p< 0.001.

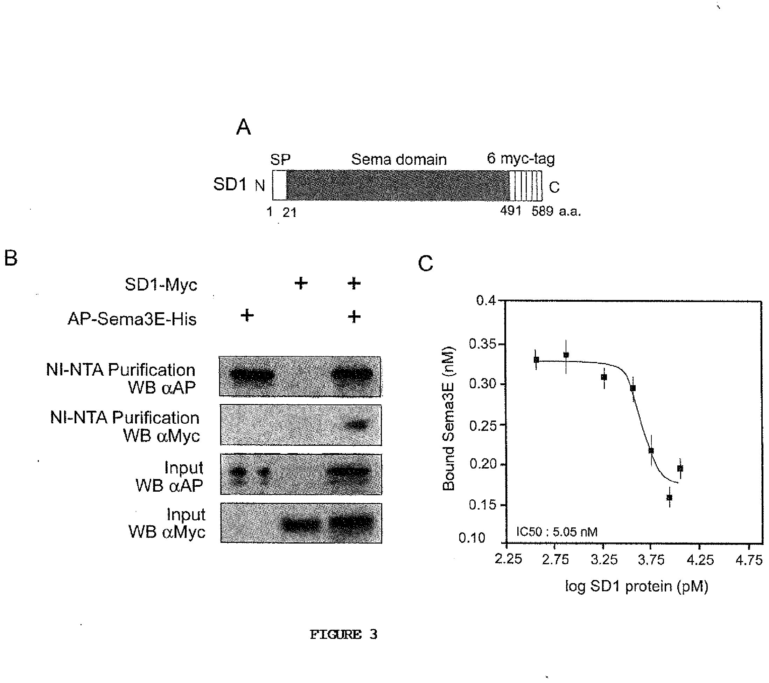

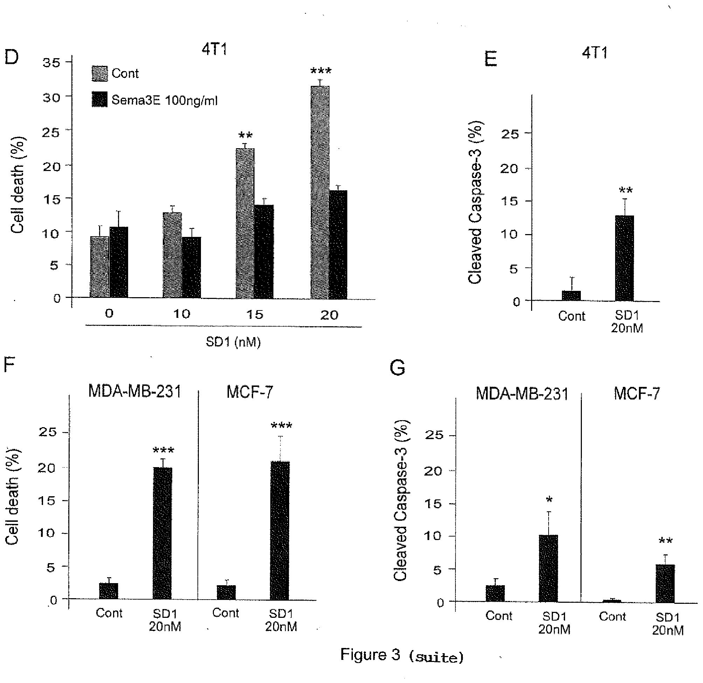

**Figure 3****. SD1 blocks Sema3E/PlexinD1 signaling *in vitro***
   (A) SD1 is composed of the immunoglobulin (Ig) k-chain secretion signal peptide, followed by the sema domain of mouse PlexinD1 protein (aa 61 to 531) fused in frame with six myc epitope tags (a.a: amino acid). (B) Co-precipitation experiments on HEK 293T cells transfected with the indicated combinations of myc-tagged SD1, Alkaline phosphatase(AP)-Histidine(His)-tagged Sema3E. SD1 can form a complex with Sema3E protein when His-tagged Sema3E is precipitated with a Nickel resin. (C) Displacement curve of Sema3E binding to PlexinD1-expressing HEK 293T cells by increasing concentrations of SD1 (from 1-12 nM). SD1 displays an antagonistic effect on Sema3E/PlexinD1 binding with an IC₅₀ of 5.05 nM. (D-G) Quantification of cell death induction by SD1 in cultures of 4T1 (D-E), MDA-MB-231 (F-G) and MCF-7 (F-G) cells measured by trypan blue exclusion (D and F) or by quantification of the percentage of cleaved Caspase-3 positive cells (E and G) in the absence or presence of exogenous Sema3E (100ng/ml). SD1 is added as conditioned medium from transfected HEK 293T cells at increasing concentration (0 to 20 nM) (D) or fixed concentration (20nM) (E-G). SD1 induces cell death in all cell lines. In 4T1 cells, SD1 acts in a dose dependent manner and addition of exogenous Sema3E is sufficient to rescue cells from death (D). Data are presented as percentage ± SEM (n = 3). *significantly different with p< 0.05. **significantly different with p< 0.01. ***significantly different with p< 0.001.
**Figure 4****.** Cultured supernatants from stable transfected cell lines, 4T1-mock or 4T1-SD1, were collected and subjected to immunoblot analysis with an anti-myc antibody (αMyc) to confirm SD1 expression in stable 4T1-SD1 cell line.
**Figure 5****. SD1 reduces the size of primary tumors and the number of lung metastasis.**
   (A) Representative images of primary tumors derived from 4T1-SD1 or 4T1-mock cells injected subcutaneously or in the mammary fat pad into syngenic Balb/c mice, 27 days post injection.
   (B-E) Tumor volume (B and D) and tumor weight (C and E) after subcutaneous (B and C) and mammary fat pad injection (D and E) of 4T1-mock or 4T1-SD1 cells. Two populations of 4T1-SD1 cells from independent clones were used in 2-3 independent experiments and consistently yielded similar results. SD1 inhibits growth of primary tumors.
   (F) Representative images of India ink-stained lungs from Balb/c mice mice carrying primary tumors of 4T1-SD1 or 4T1-mock cells, harvested 27 days after subcutaneous or mammary fat pad injections. The arrows point to the metastatic nodules in the lung.
   (G-I) Total number of visible superficial (G and I) or internal (H) metastatic nodules from the lung of individual mice, were counted after observation under a dissection microscope. Two populations of independent 4T1-SD1 cells from independent clones were used in 2-3 independent experiments and consistently yielded similar results. SD1 expression inhibited the ability of 4T1 cells to metastasize from the subcutaneous tumor (G and H) or mammary tumors (I) to the lung.
   *significantly different with p< 0.05, ***significantly different with p< 0.001. Scale bar 4.5 mm (A). Scale bar 3.8 mm (F).

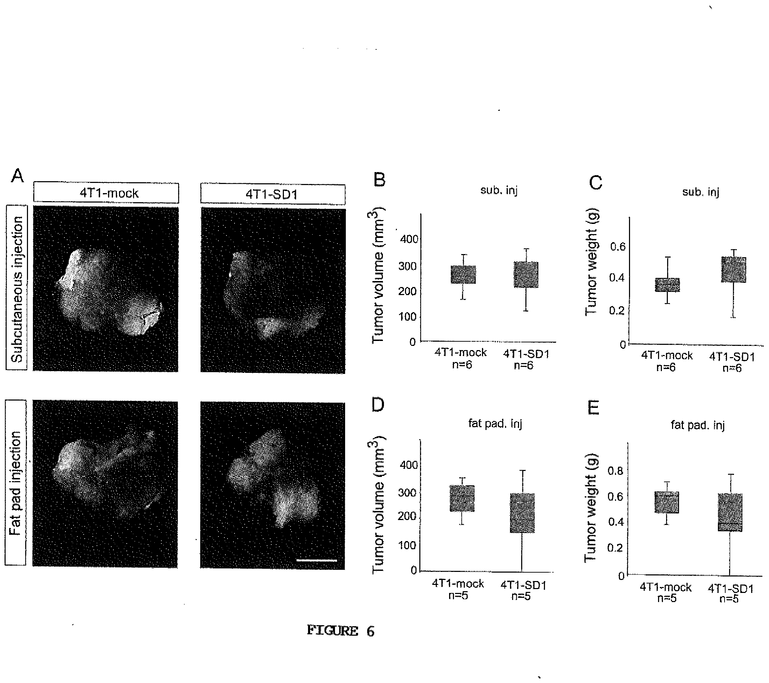

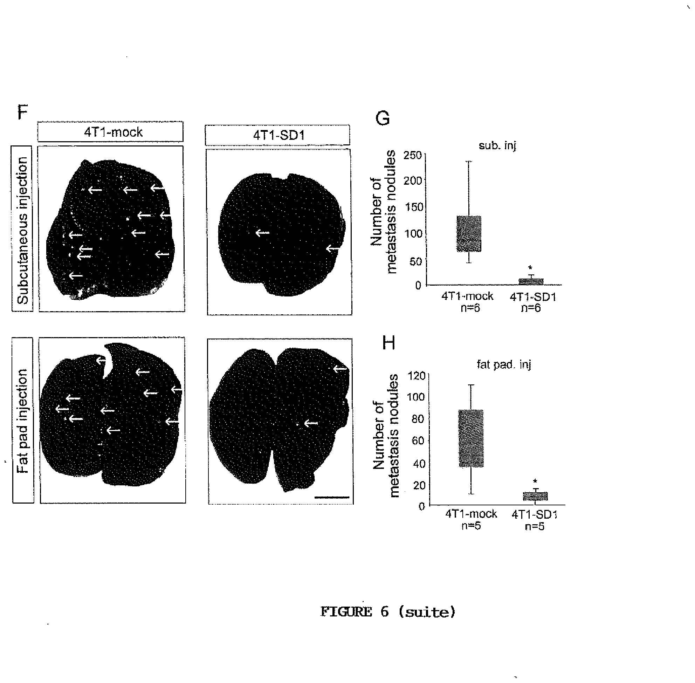

**Figure 6****. SD1 expression inhibits lung metastasis independently of its effect on primary tumor growth**
   (A) Representative images of primary tumors derived from 4T1-SD1 or 4T1-mock cells injected subcutaneously or in the mammary fat pad into syngenic Balb/c mice. Primary tumors were collected when tumors had reached an average volume of 300 mm³ (4T1-mock/subcutaneous: 46 ± 4 dpi (day post-injection); 4T1-SD1 /subcutaneous: 60 ± 3 dpi; 4T1-mock/fat pad: 31 ± 3 dpi; 4T1-SD1 /fat pad: 50 ± 6 dpi).
   (B-E) Tumor volume (B and D) and tumor weight (C and E) corresponding to tumors described in (A). Primary tumors display similar size.
   (F) Representative images of the India ink-stained lungs from mice carrying primary tumors of similar size of 4T1-SD1 or 4T1-mock cells injected subcutaneously or in the mammary fat pad of syngenic Balb/c mice. The arrows point to the metastatic nodules in the lung.
   (G and H) Total number of visible superficial metastatic nodules in individual mice was counted under a dissection microscope. The average number of metastatic nodules per lung in animals transplanted subcutaneously (G, n=6/4T1-mock and n= 6/4T1-SD1) and in mammary fat pad (H, n=5/4T1-mock and n= 5/4T1-SD1) was smaller with 4T1-SD1 cells than with 4T1-mock cells, despite similar sized primary tumors. *significantly different with p< 0.05. Scale bar 4.5 mm (A). Scale bar 3.8 mm (F).

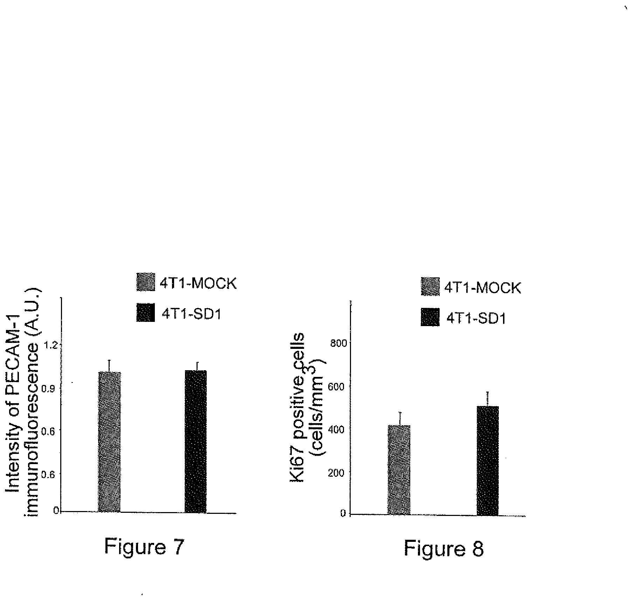

**Figure 7****.** Graph illustrating the mean pixel intensity of PECAM-1 staining within primary tumors.
**Figure 8****.** Graph indicating the number of Ki67 positive cell per mm³.

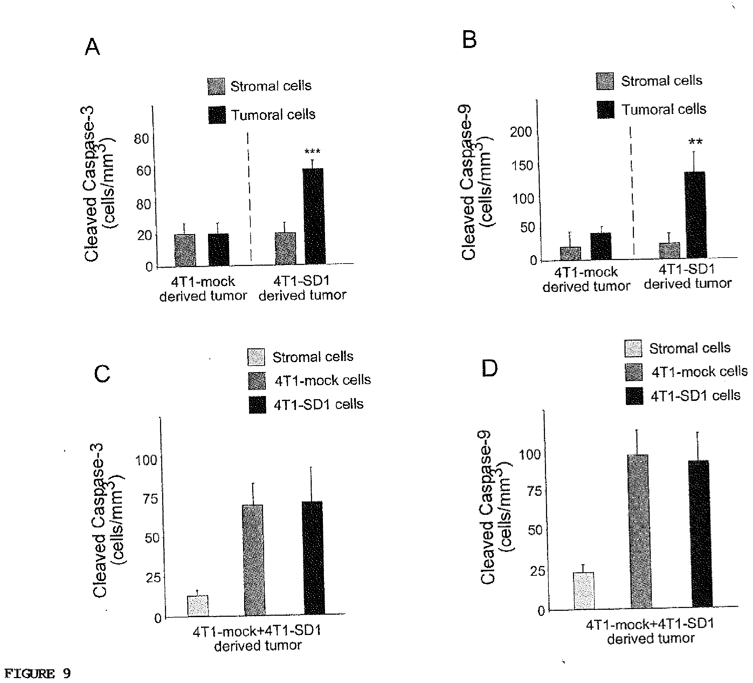

**Figure 9****. SD1 induces tumor cell death *in vivo.*** Immunolabeling of cleaved Caspase-3 and cleaved Caspase-9 on primary tumor sections from 4T1-mock or 4T-SD1 cells injected subcutaneously into syngenic Balb/c mice, 27 days post injection. 4T1 cells were done and visualized by expression of the myristoylated Cherry protein (mCherry) (data not shown).
   (A and B) Quantification of the immunostaining. SD1 expression significantly increased the number of cleaved Caspase-3 positive (A) and of cleaved Caspase-9 positive (B) 4T1 cells but not that of stromal cells.
   (C and D) Quantification of cleaved Caspase-3 and cleaved Caspase-9 positive cells on primary tumor sections derived from coinjection of 4T1-mock and 4T-SD1 cells (ratio 1:1) subcutaneously into syngenic Balb/c mice 27 days post injection. SD1 expression significantly increased cell death of 4T1-SD1 as well as 4T1-mock co-injected cells, but not that of stromal cells.
   For Figures 7-8, values show the average ± SEM from 2-4 tumors per experimental group; at least 3 sections and 10 fields were analyzed for each tumor. **significantly different with p< 0.01, ***significantly different with p< 0.001.).

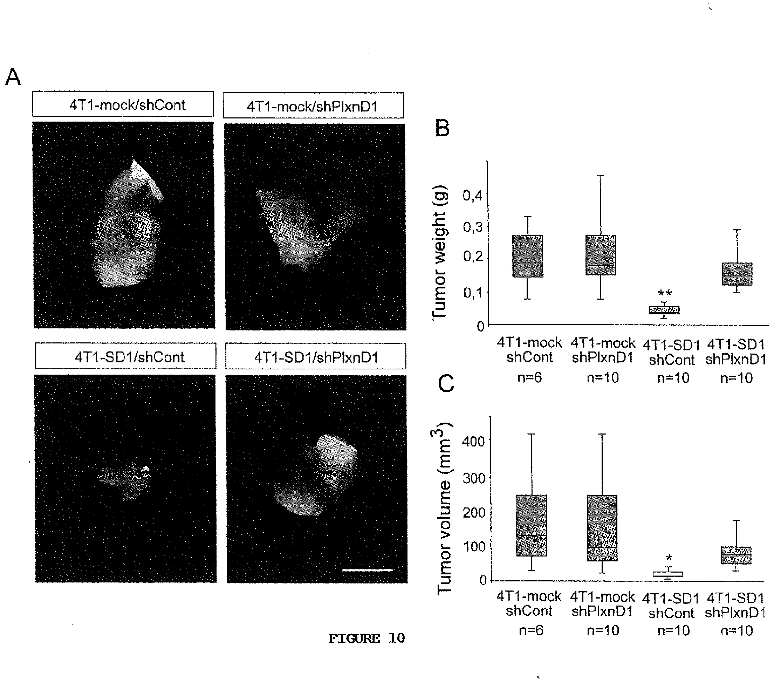

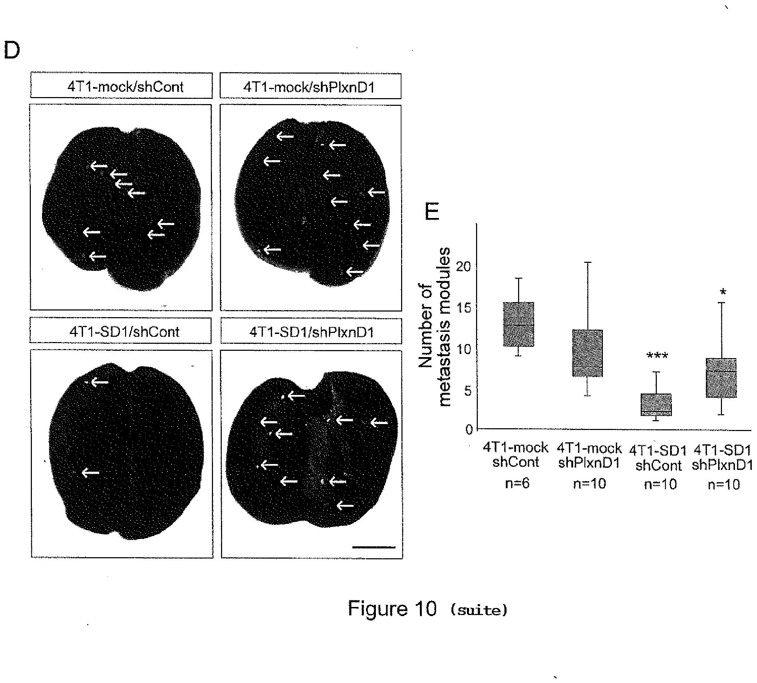

**Figure 10****. SD1 inhibitory effect on tumor progression requires PlexinD1 on tumor cells.**
   (A) Representative images of primary tumors derived from 4T1-mock or 4T1-SD1 cells transfected with shRNA control or shRNA to mouse PlexinD1 and injected subcutaneously into syngenic Balb/c mice, 27 days post-injection.
   (B and C) Volume (B) and weight (C) of tumors described in (A). Knockdown of PlexinD1 blocks the inhibitory effect of SD1 on primary tumors growth.
   (D) Representative images of India ink-stained lungs from Balb/c mice mice carrying primary tumors from 4T1-mock or 4T1-SD1 cells transfected with shRNA control or shRNA to mouse PlexinD1, harvested 27 days after subcutaneous injection. The arrows point to the metastatic nodules in the lung.
   (E) Total number of visible superficial metastatic nodules in individual mice was counted under a dissection microscope. Knockdown of PlexinD1 blocks the inhibitory effect of SD1 on metastasis to the lungs.
   *significantly different with p< 0.05. **significantly different with p< 0.01. ***significantly different with p< 0.001. Scale bar 4.5 mm (A). Scale bar 3.8 mm (D).

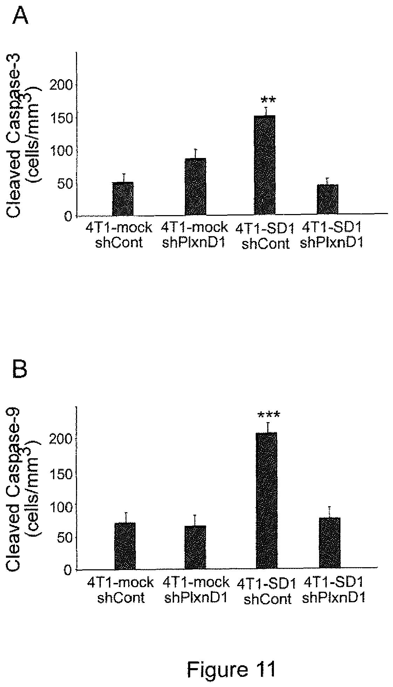

**Figure 11****. Caspase mediated cell death induced by SD1 requires PlexinD1 on tumor cells.** Immunolabeling of cleaved Caspase-3 (A-H) and cleaved Caspase-9 (F-P) on primary tumor sections derived from 4T1-mock or 4T1-SD1 cells transfected with shRNA control or shRNA to mouse PlexinD1, injected subcutaneously into syngenic Balb/c mice 27 days post-injection were done and 4T1 cells labeled by the mCherry protein (not shown). (A and B) Quantification of Caspase-3 immunostaining and Caspase-9 immunostaining. The increase of the percentage of cleaved Caspase-3 and cleaved Caspase-9 positive cells mediated by SD1 is blocked by knocking-down PlexinD1 expression. Values show the average ± SEM from 2-4 tumors per experimental group; at least 3 sections and 10 fields were analyzed for each tumor. **significantly different with p< 0.01, ***significantly different with p< 0.001.

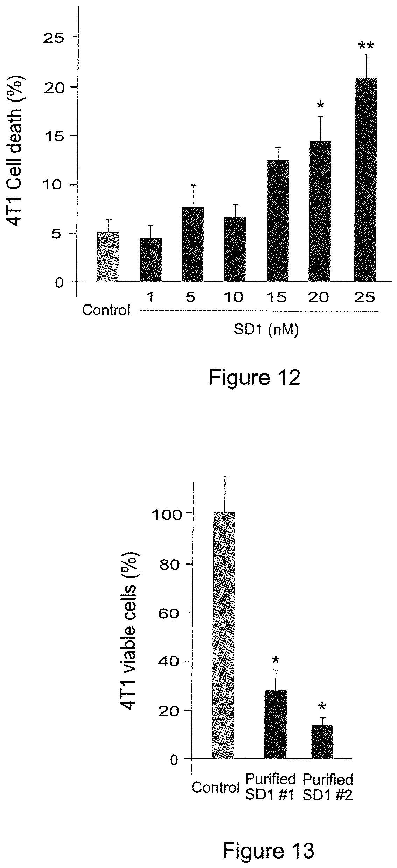

**Figure 12****.** Quantification of cell death induction by SD1 in cultures of 4T1 cells measured by trypan blue exclusion. SD1 is added as conditioned medium. Data are presented as percentage ± SEM (n = 3).
**Figure 13****.** Quantification of cell death induction by SD1 in cultures of 4T1 cells measured by calcein assay. SD1 is added as purified protein (two different purifications have been tested #1-#2). Data are presented as percentage ± SEM (n = 3).

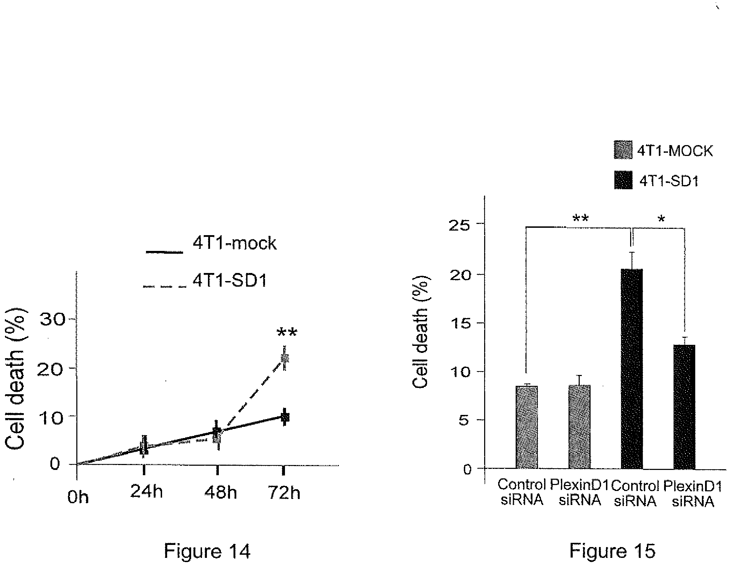

**Figure 14****.** Stable transfected cell lines, 4T1-mock or 4T1-SD1, were grown in culture for three days. Cell death was evaluated daily, by trypan blue-exclusion. Data represent the average of triplicates ± SEM. Similar results were obtained analyzing different 4T1-mock or 4T1-SD1 clones.
**Figure 15****.** Stable transfected cell lines, 4T1-mock or 4T1-SD1, were transfected with control siRNA or siRNA to mouse PlexinD1. Cell death was evaluated by trypan blue exclusion assay.

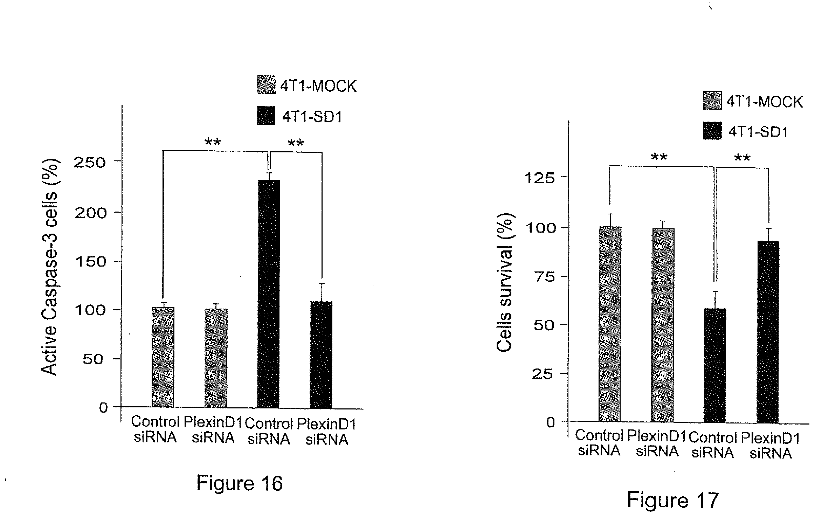

**Figure 16****.** Stable transfected cell lines, 4T1-mock or 4T1-SD1, were transfected with control siRNA or siRNA to mouse PlexinD1. Cell death was evaluated by caspase-3 activity measurement. Knockdown of PlexinD1 causes significant decrease of SD1 cell-death effect.
**Figure 17****.** Stable transfected cell lines, 4T1-mock or 4T1-SD1, were transfected with control siRNA or siRNA to mouse PlexinD1. Cell survival was evaluated by MTT assay.
   For Figures 12-17, datas are presented as average ± SEM (n = 3) and are normalized to 100% for values obtained in control conditions (Figures 14 and 15). *significantly different with p< 0.05. **significantly different with p< 0. 01.

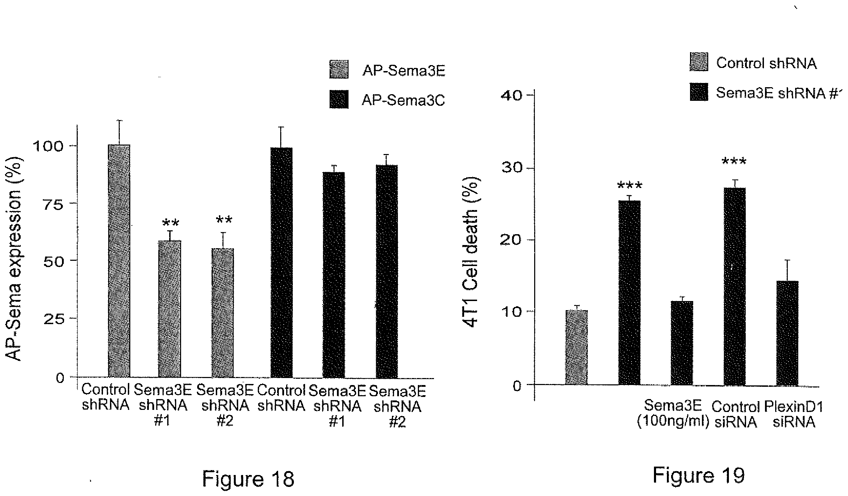

**Figure 18****.** Controls for specificity of knockdown of mouse Sema3E by shRNAs. Hek293T cells were electroporated with an AP-Sema3E or AP-Sema3C expression vector to produce AP-fusion protein in the medium together with control shRNA or Sema3E shRNA1 and 2. Targeted shRNAs led to diminution of AP-Sema3E expression in the culture medium without affecting AP-Sema3C production.
**Figure 19****.** 4T1 cells were transfected with control shRNA or shRNA1 to mouse Sema3E together with control siRNA or siRNA to mouse PlexinD1 in presence or absence of exogenous Sema3E (100ng/ml). Cell death was evaluated by trypan blue-exclusion assay. Exogenous Sema3E rescues 4T1 cell-death induced by knockdown of Sema3E. Knockdown of PlexinD1 abolishes the effect of Sema3E knockdown.

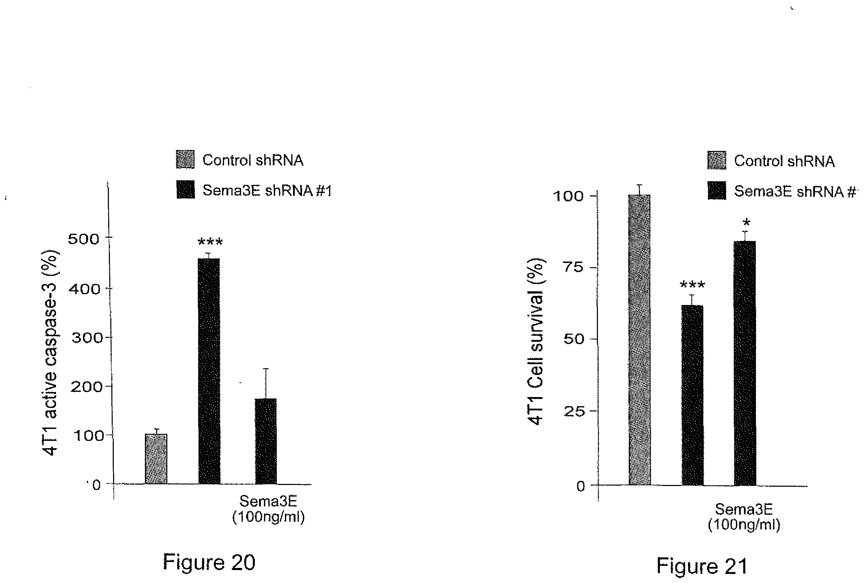

**Figure 20****.** Quantification of the results of immunostaining of active Caspase-3 on 4T1 cell line. 4T1 cells were transfected with control shRNA or shRNA1 to mouse Sema3E in presence or absence of exogenous Sema3E (100ng/ml). Lowering Sema3E level activates caspase-3.
**Figure 21****.** 4T1-mock or 4T1-SD1 cells were transfected with control shRNA or shRNA1 to mouse Sema3E. Cells survival was evaluated by MTT assay. Knockdown of Sema3E causes significant decrease in cell survival.
   For Figures 18-21, *significantly different with p< 0.05. **significantly different with p< 0.01. ***significantly different with p< 0.001.

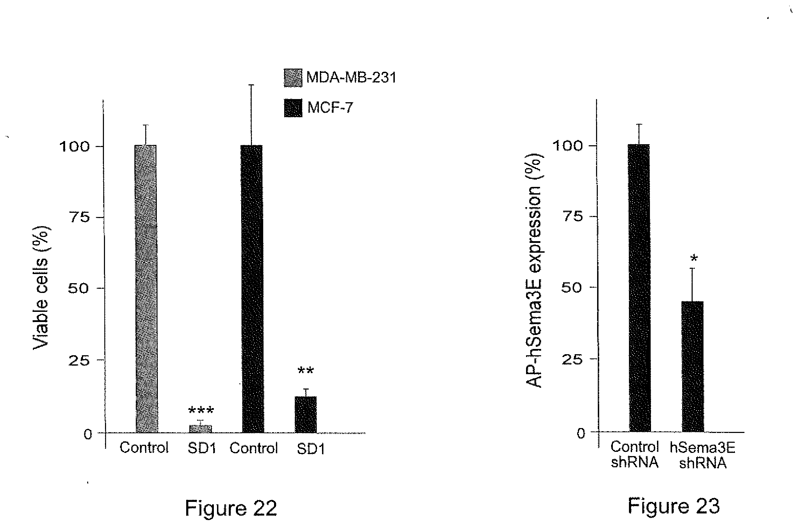

**Figure 22****.** MDA-MB-231 or MCF-7 tumor cells were cultured in the presence or absence of SD1 conditioned medium (15 nM). After three days, cells are stained with calcein to measure cell survival. SD1 expression diminishes significantly tumor cells viability.
**Figure 23****.** Controls for specificity of knockdown of human Sema3E by shRNA. Hek293T cells were electroporated with a human AP-Sema3E (AP-hSema3E) expression vector to produce AP-fusion protein in the medium together with control shRNA or hSema3E shRNA. Targeted shRNAs led to diminution of AP-hSema3E expression.

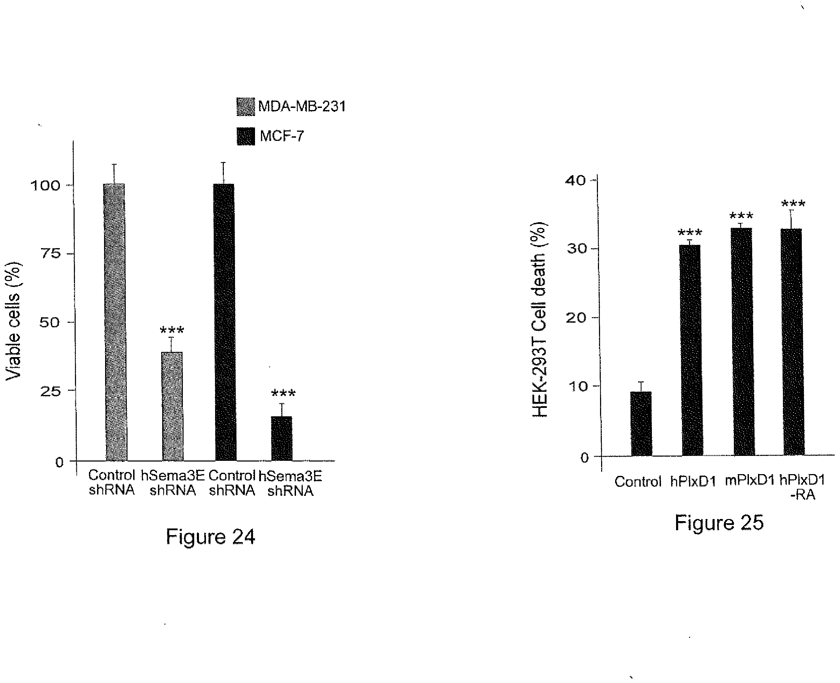

**Figure 24****.** MDA-MB-231 or MCF-7 tumor cells were transfected with control shRNA or shRNA to human Sema3E. Knockdown Sema3E significantly inhibits viability of human tumor cells.
   For Figures 22-24, *significantly different with p< 0.05. **significantly different with p< 0.01. ***significantly different with p< 0.001.
**Figures 25-26****:** Cell death of HEK 293T cells transfected with human PlexinD1 (hPlexinD1), mouse PlexinD1 (mPlexinD1) or hPlexinD1 mutated in R-RAS GAP activity domains (hPlexinD1-RA) compared to HEK 293T cells transfected with a mock vector, was measured by trypan blue-exclusion assay (25) or by quantification of the percentage of cleaved Caspase-3 positive cells (26). Exogenous expression of PlexinD1 induces cell death, without requiring its GAP activity.

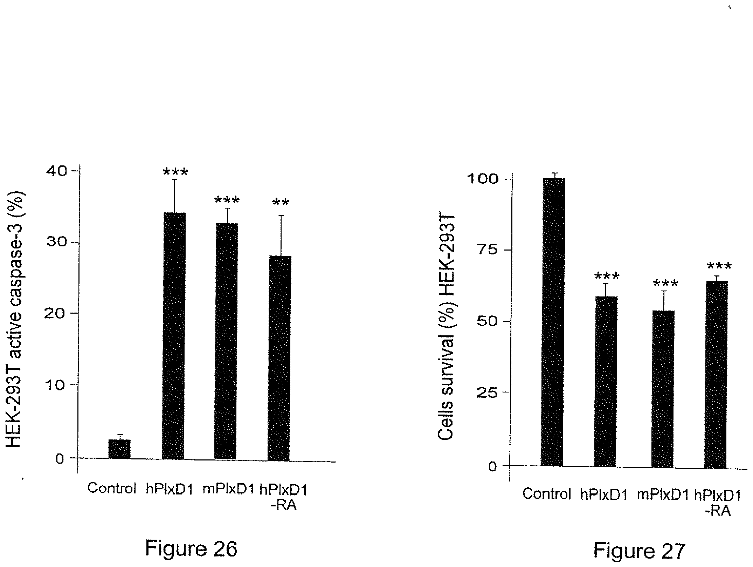

**Figure 27****.** Hek293 cells were transfected with hPlexinD1, mPlexinD1 or hPlexinD1-RA. Cell survival was evaluated by MTT assay. Expression of PlexinD1 causes significant decrease in cell survival.

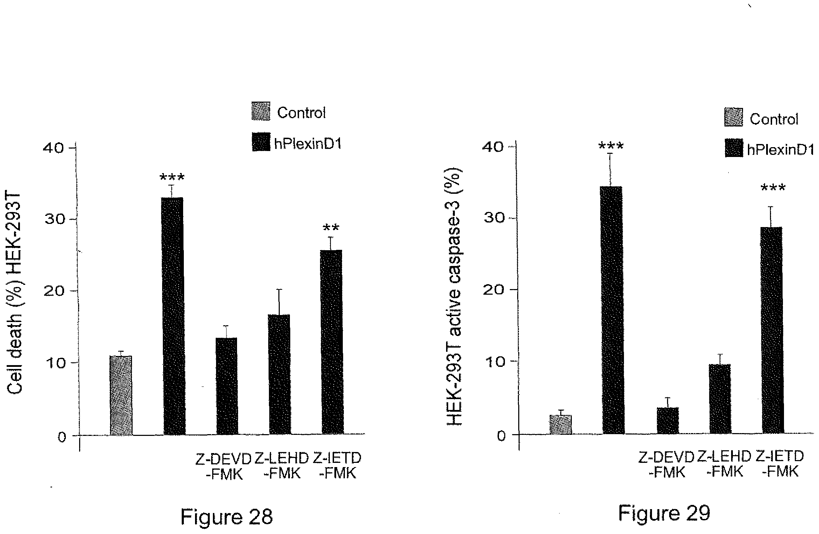

**Figures 28-29****:** HEK 293T cells transfected with an expression control vector or hPlexinD1 were cultured in absence or presence of different caspase inhibitors at 10µM (caspase-3 (z-DEVD-fmk) and capspase-9 (z-LEHD-fmk), caspase-8 (z-IETD-fmk)). Cell death was measured by trypan blue exclusion-assay (28) or by quantification of the percentage of cleaved Caspase-3 positive cells (29). Activation of caspase-9 and caspase-3 is required for PlexinD1-mediated cell death.

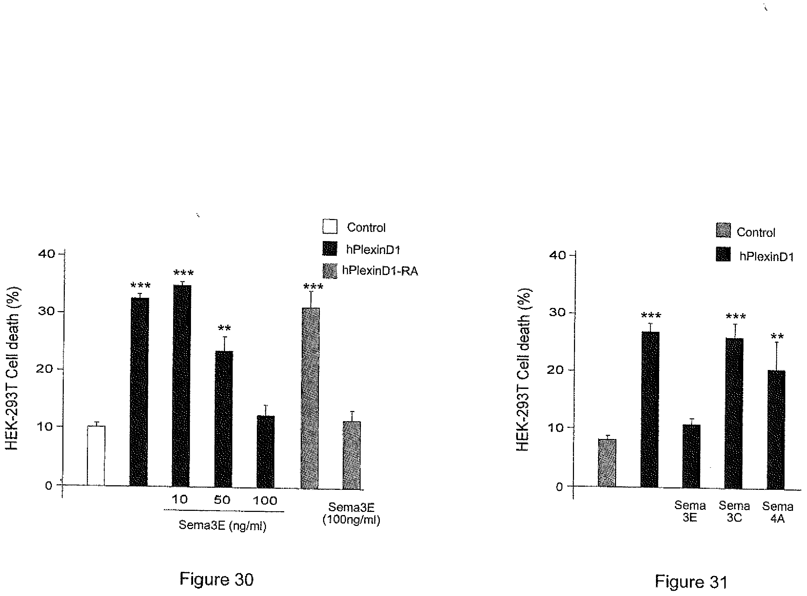

**Figure 30****:** HEK 293T cells transfected with expression vectors for hPlexinD1, hPlexinD1-RA or a control vector were cultured in the absence or presence of different concentration of Sema3E (10 to 100 ng/ml). Sema3E ligand dose-dependently inhibits cell death induced by PlexinD1 expression.
**Figure 31****: HEK** 293T cells transfected with an expression vector for hPlexinD1 together with expression vector coding for Sema3E, Sema3C or a soluble form of Sema4A. Unlike Sema3E, Sema3C and Sema4A do not inhibit cell death induced by PlexinD1 expression.
   For figures 25-31, **significantly different with p< 0.01. ***significantly different with p< 0.001.

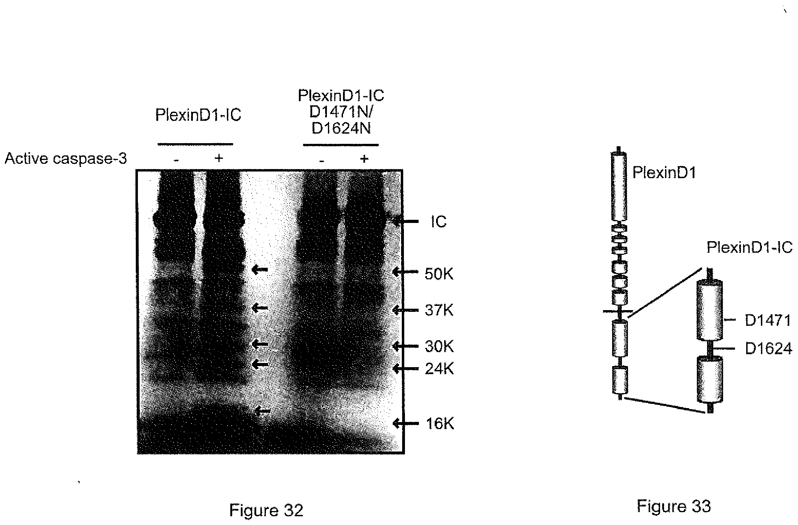

**Figure 32****.** PlexinD1 is cleaved by caspase 3 *in vitro.* The *in vitro* translated intracellular domain of PlexinD1 (PlexinD1-IC) and PlexinD1-IC D1471N/D1624N were incubated in the absence of caspase or with purified active caspase-3 (0.5 µM). An autoradiographie is shown. The PlexinD1-IC D1471N/D1624N mutant is not cleaved by caspase-3.
**Figure 33****.** Scheme representing the position of the aspartic acid residues 1471 and 1624 in PlexinD1 intracellular domain.

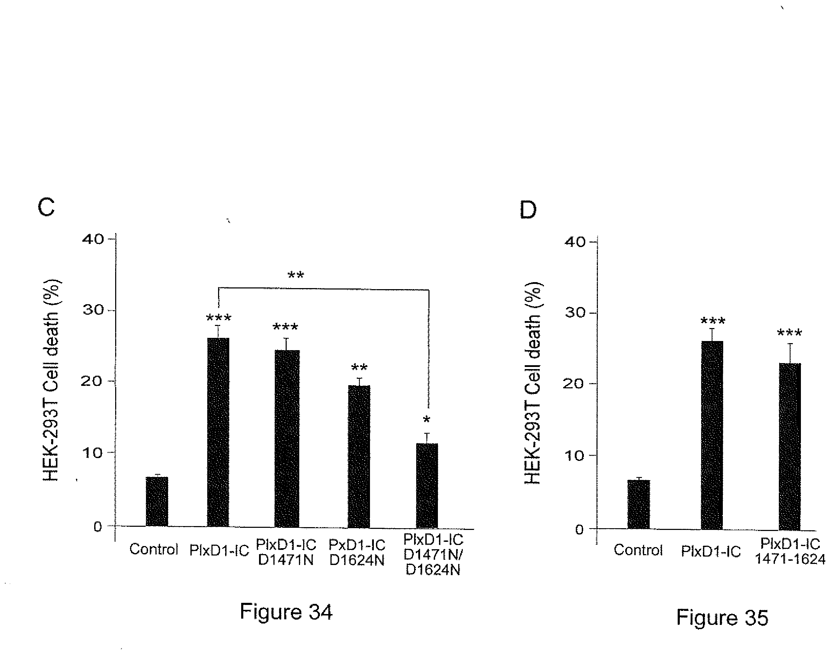

**Figures 34 and 35****.** Hek293T cells were transfected with plasmids encoding PlexinD1-IC, PlexinD1-IC mutated on D1471 and/or D1624 (Figure 34), or PlexinD1 1471-1624 fragment (Figure 35). Cell survival was evaluated by trypan blue-exclusion assay. PlexinD1 1471-1624 fragment is sufficient to induce cell death.
   *significantly different with p< 0.05. **significantly different with p< 0.01. ***significantly different with p< 0.001.

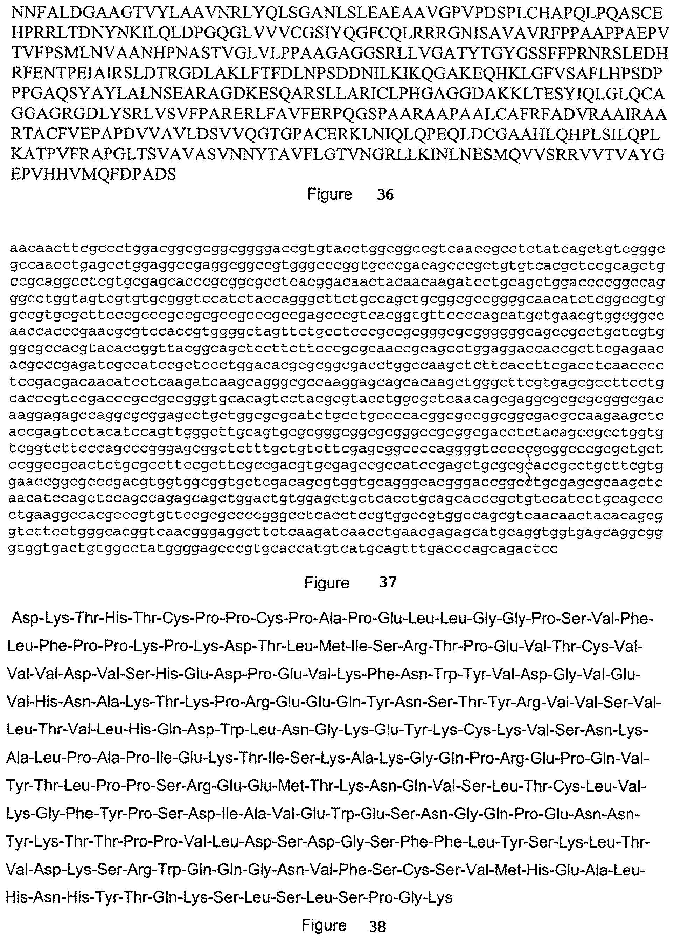

**Figure 36****.** Amino acid sequence of the sema domain of PlexinD1: SEQ ID NO:1
**Figure 37****.** Nucleotide sequence of the sema domain of PlexinD1: SEQ ID NO:2
**Figure 38****.** Amino acid sequence of Fc: SEQ ID NO:3

### EXPERIMENTAL PROCEDURES

### Cell lines and cell culture

4T1, HEK-293T, MDA-MB-231, MCF-7 and U-87 MG cells lines were obtained from American Type Culture Collection (ATCC). Cultures were performed following the standard culture condition from ATCC.

### RT-PCR gene expression

Total RNA from 4T1 cells was isolated using RNeasy Protect Mini Kit (74104, Qiagen) according to manufacturer's instructions. cDNA preparation was performed according to standard procedures, using Quantitect Reverse transcript Kit (205311. Qiagen) and oligo-dT primers. PCR were performed using the following primers:
PlexinD1:
   5'-TGTATGGGCGAATAACTCACTG-3' (**SEQ ID NO:4**)
      and
   5'-TCATTCCAAACACAGAGGTGAC-3' (**SEQ ID NO:5**)
Sema3E:
   5'-GGGGACCACTTTGTACAAGAAAGCTGGGTCCTAGGAGAGCAGCGTGTGCCTGGGCA-3'(**SEQ ID NO:6**)
      and
   5'-GGGGACAAGTTTGTACAAAAAGCAGGCTTCGCGAACCCCTCCTACCCCAGGCT-3' (**SEQ ID NO:7**)
GAPDH:
   5'-ACCCAGAAGACTGTGGATGG-3' (**SEQ ID NO:8**)
      and
   5'-GGAGACAACCTGGTCCTCAG-3' (**SEQ ID NO:9**)

### Immunohistochemistry

Immunohistochemistry on primary tumor sections and cell cultures were carried out following standard procedures with the following antibodies : rat anti-PECAM (1:40, 553370, BD Bioscience Pharmingen), goat anti-PlexinD1 (1:100, AF4160, R&D System Research), goat anti-Sema3E (1:100, YRG01, R&D System Research), rabbit anti-active caspase-3 (1:200, 9661S, Cell Signaling), rat anti-Ki67 (1:25, M7249, DAKO), Alexa Fluor 555-conjugated donkey anti-goat IgG (1:500, Interchim Molecular Probes), Alexa Fluor 488-conjugated donkey anti-goat IgG (1:500, Interchim Molecular Probes), Alexa Fluor 488-conjugated donkey anti-rat IgG (1:500, Invitrogen Molecular Probes), and Alexa Fluor 488-conjugated donkey anti-rabbit IgG (1:500, Invitrogen Molecular Probes).

### Quantitative fluorescence determinations of blood vessel density

PECAM-1-stained primary tumor sections were imaged with a confocal microscope (Zeiss Apotom, Axioimager Z1) equipped with 20x Plan-NEOFLUAR objective. Parameters of time interval and gain setting on the camera were adjusted so the brightest areas did not reach saturation, and the same gain and time interval was used to capture all images of any particular staining was used for image analysis. The immunofluorescence intensity within different areas of the tumors were measured using the ImageJ software in 10 different pictures for each tumor.

### Plasmid construction and site-directed mutagenesis.

The Sema domain of PlexinD1 (SD1) was amplified by PCR from a vector that contains PlexinD1 cDNA. Primers:
Forward 5'-CCATCGATTAATGGCT CGTCGCGCGCGGGC-3' (**SEQ ID NO:10**),
Reverse 5'-GGATCGATGATCCATGGGGTCAAACTGCAT-3' (**SEQ ID NO:11**)**.**

The sequence coding for the IgK secretion signal peptide was amplified by PCR from the APtag-5 vector. Primers:
Forward 5'-CCATCGATTAATGGAGACAGACACACTCCT-3' (**SEQ ID NO:12**),
Reverse 5'-AGTTAAAACCGTACGCGTCACCAGTGGAAGGT-3' (**SEQ ID NO:13**).

The total insert is ∼1.5kb in length and was subcloned in the Clal clonage site in the pCS2-Myc vector

The plasmid PlexinD1-IC was constructed by inserting the intracellular domain of PlexinD1 into pDONR221 (Invitrogen) using the polymerase chain reaction (PCR) primers from a vector that contains PlexinD1 cDNA. Next the insert was subcloned in the pDest26, pDest17 or pcDNA3.1/nV5-DEST (Invitrogen) vectors.

The mutation of PlexinD1-IC D1471N, PlexinD1-IC D1624N, and PlexinD1-IC D1471N/D1624N was obtained by PCR from the PlexinD1-IC pDONR221 vector, using: mutation D1471N primers:
forward 5'-GGACCTCATTAACGCCTCGGCCGCCAAGAACCCCAA-3' (**SEQ ID NO:16)**
reverse 5'-CGGCCGAGGCGTTAATGAGGTCCACCAGCAGCTCCTTC-3' (**SEQ ID NO:17)** mutation D1624N primers :
   forward 5'-GGACCTGGACAACACCTCAGTGGTGGAAGACGGCC-3' (**SEQ ID NO:18)**
   reverse 5'-CCACTGAGGTGTTGTCCAGGTCCCGAAGGATGTAGCTC-3' (**SEQ ID NO:19)** then subcloned in pcDNA3.1/nV5-DEST vector (Invitrogen).

The D1471N/D1624N fragment was amplified by PCR from PlexinD1-IC vector. Primers:
Forward 5'-GGGGACAAGTTTGTACAAAAAAGCAGGCTTCGCCTCGGCCGCCAAGAACC CCAAG-3' (**SEQ ID NO:20**),
Reverse 5'-GGGGACCACTTTGTACAAGAAAGCTGGGTCCTAGTCGTCCAGGTCCCGAA G GATGTA-3' (**SEQ ID NO:21)**
then subcloned in the pDONR221 (Invitrogen) before subcloned in pDest26 (Invitrogen).

The mutation of hPlexinD1-RA, was obtained by PCR from the hPlexinD1 expression vector.
Mutation LRR-1482-LAA primers:
forward 5'-CATGCTGGCCGCCACAGAGTCTGTGGTGGAGAAGATGCTCACC-3' (**SEQ ID NO:22),**
reverse 5'-CTCTGTGGCGGC CAGCATGAGCTTGGGGTTCTTGGCGGCC-3' (**SEQ ID NO:23),**
Mutation LRF-1769-LAF primers :
forward 5'- CCTCTCGCCTTCTGGGTGAACATCCTGAACCCCCAGT-3' (**SEQ ID NO:24),**
reverse 5'-CCAGAAG GCGAGAGGAAGGCTGTTGGTCTTCCAGATGTGT-3') (**SEQ ID NO:25).**

### Production and purification of SD1

HEK293T cells were transfected with a vector encoding SD1 or a control pCS2-Myc vector using Lipofectamine plus (Invitrogen). Conditioned medium was collected 3 days after transfection, concentrated using 30,000 KDa cut-off Centricon devices (Millipore) and the concentration of SD1 was quantified by Western blot analysis.

For purification of SD1, one ml of the concentrate was incubated with 100 µl of agarose immobilized rabbit anti-C-myc beads (Gentaur, Brussels, Belgium) for 1 hour at 4 °C. Beads were washed 10 times with 1 ml of PBS and SD1 eluted by adding 0.4 ml of 0.1 M citric acid (pH 2.4). Immediately after spinning and collection of supernatant, 40 µl of 1M Tris (pH 9) was added to neutralize the pH. 0.2 ml of SD1 was mixed with 20 volumes of OptiMEM and concentrated down to 0.36 ml used for treatment of 4T1 cells. Control conditions consisted of 4T1 cells treated with medium from non-transfected HEK293T cells identically processed.

### Immunoprecipitation and Western Blotting

HEK293T cells were transfected with AP-Sema3E-his and/or equal amounts of SD1 vectors. Two days after transfection, cells were lysed in Ramesh solution (Tris 50nM pH8, NaCl 150nM, Sodium Fluoride 50nM, EDTA 2mM, Orthovanadate 0,1mM, NP40 0.5%, antiprotease (11697498001. Roche)). The cell lysates were immunoprecipitated using NI-NTA Agarose (30210. Qigagen) for his-tagged protein precipitation. Complexes were separated by electrophoresis and electrotransferred onto membrane (Immobilon- P, Millipore). Membranes were incubated with one of the following antibodies: mouse anti-Myc (1:500, 9E10, Sigma), rabbit anti-AP (1:2000, sc-28904, Santa Cruz). After incubation with HRP conjugated secondary antibodies, signals were detected with enhanced chemoluminescence system (GE Healthcare, RPN2132).

### Production of Semaphorin proteins

Production of mouse AP-Sema3C, AP-Sema3E and AP-Sema3F was performed as described previously (Chauvet et al., 2007). The AP-Sema3F construct was a gift from A. Kolodkin. Concentrations of AP-tagged proteins were measured using the SIGMAFAST™ p-Nitrophenyl phosphate Tablets kit (N1891, Sigma).

### Dissociated neuronal cultures

Brains from wild-type CD1 mice were dissected to extract subiculum and ventrolateral cortex at E17.5, or neo-cortex and hippocampus at E15.5. Neurons were dissociated, electroporated with a GFP-pCAGGS vector alone or together with expression vector encoding SD1 and cultured as described (Chauvet et al, 2007) with 5nM Sema3E-AP or 10nM Sema3C-AP or 10nM Sema3F-AP. After 2-3 days in vitro, cultures were fixed, immunostained with rabbit anti-GFP antibody (1:500, Torrey Pines). Secondary antibody was Alexa Fluor 488-conjugated goat anti-rabbit (1:400, Interchim Molecular Probes). The growth effect was quantified as described (Chauvet et al, 2007).

### Establishing stable cell lines

A plasmid vector encoding myristoylated Cherry (gift from X. Morin) was co-transfected together with the SD1 vector or control pCS2-Myc vector into 4T1 cells using a LipofectAMINE plus protocol (Invitrogen Life Technologies). After transfection, the cells were grown for 2 days in a nonselective growth medium that was then replaced with a selection medium containing geneticin (10131-027, GIBCO). After 2 weeks, individual colonies were isolated and selected for cherry expression under an inverse microscope (Zeiss observerD1) and SD1 production by Western blot using anti-myc antibody (1:500, 9E10, Sigma). For each condition, two independent cell-derived clones were subsequently expanded in selection medium for use in experiments.

### Animal models and in vivo procedures

*In vivo* studies were conducted in 6-8 weeks old BALB/c ByJ female mice (Charles River Laboratories France). 4.5×10⁵ 4T1-mock or 4T1-SD1 cells were injected into the right posterior flank or into the mammary fad pad of anaesthetized animals. Tumor size was measured externally every two days using a calliper, and the width (A) and length (B) of the developing tumor was converted to volume using the equation V=0.52xA²xB. Mice were sacrificed 27 days after injection or when the tumor volume was equal to 300 mm³. Tumors and lungs were dissected and fixed in Destainer Solution (49% formaldehyde 37%, 49% Ethanol 70% and 2% Acetic Acid Glacial) overnight at 4°C then weighted. Superficial pulmonary metastases were contrasted by black India-ink airways infusion, and counted on dissected lung lobes under a stereoscopic microscope.

### Knock down of gene expression by RNA-interference.

The expression of mouse Sema3E, was silenced in tumor cells by expressing two different gene-targeted small hairpin RNA from Sigma
(shRNA1): 5'- CCGGGCTGCTGAAAGTAATCACAATCTCGAGATTGTGATTACTTTCA GCAGCTTTTTG -3' (**SEQ ID NO:26),**
or (shRNA2): 5'- CCGGCCTTGTCTATTCCCTGAACTTCTCGAGAAGTTCAGGGAATA GACAAGGTTTTTG -3' (**SEQ ID NO:27).**

siRNA PlexinD1 or siRNA control from Dharmacon RNA Technologies were used to knockdown mouse PlexinD1 (Chauvet et al. 2007).

### Cell death and cell viability analysis.

Cell death/viability was determined by trypan blue dye exclusion, MTT cell viability assay, calcein assay, and anti-active caspase-3 immunoreactivity. In some experiments, cells were seeded in multiple 24-well plates (5 × 10⁴ cells/well) for 24 h then transfected with different vectors using Lipofectamine Plus (Invitrogen). The different analysis occurred after 48 h (HEK-293T cells) or 72 h (4T1 cells) of serum starvation. In some experiments, cells were cultured in the presence of 10µM caspase inhibitors: caspase-3 inhibitor II (Z-DEVD-FMK, 264155, VWR), caspase-8 inhibitor II (Z-IETD-FMK, 218759, VWR), and caspase-9 inhibitor II (Z-LEHD-FMK, 218776, VWR).

For trypan blue exclusion experiments, total cells (adherent and floating) were collected by mechanical detachment and medium collection. Cells were pelleted by slow centrifugation and resuspended in serum-free medium. Trypan blue (0.4%) was added in a ratio of 1:1 to the cell suspension, and the percentage of cell death was measured in a hemacytometer by counting, in a blinded fashion and within a given volume, the number of blue (dead) cells and comparing this number to the total number of cells in this same volume.

For MTT assays, the cells were cultured in DMEM medium containing 0.1% FBS. To reveal viability, the cells were incubated with 100µl of 3-(4,5-dimethylthiazol-2-yl)-2,5-diphenyltetrazolium bromide (MTT) dye solution (M-2128, Sigma Aldrich) for 4 h at 37°C. The indicator product, formazan, was resuspended in DMSO and measured spectrophotometrically at 570 nm by Tristar LB941 (Berthold).

For caspase staining, after immunostaining with anti-active caspase-3 antibody, the percentage of labelled cells was estimated by comparing the number of active caspase-3-stained cells with the total number of cells present on a given surface.

For assessing cell viability using calcein, cells were plated in OptiMEM medium in 96-well black plates (Greiner Bio-one, Courtaboeuf, France). After 3-6 days in culture, live cells were stained with 0.5µg/ml green calcein-acetoxymethyl ester (Molecular Probes, Eugene, OR) for 40 minutes at 37 °C, and counted using a flash cytometer (Trophos, Marseille, France).

### In vitro translation and caspase-mediated cleavage reactions.

PlexinD1-IC and PlexinD1-IC D1471N/D1624N were transcribed using T7 polymerase, then translated using the TNT system (Promega) in the presence of 50µCi [35S] methionine (NEG-772, PerkinElmer) for 2 h at 30°C. Translations were incubated for 2 h in 20mM PIPES, 100mM NaCl, 1% sucrose, 10mMdithiothreitol and 0.1mM EDTA, pH 7.2, at 37°C in the presence of 0.5 µM active caspase-3. Proteins were separated by electrophoresis on 4%-20% gradient gel (WT4202BOX, Invitrogen). Gel was dried 2 h at 80°C. Signals were detected using Kodak BioMAX MR films (8929655, Kodak).

### RESULTS

### PlxnD1 and Sema3E expression in human cancers (Figure 1)

Sema3E was initially identified from tumor cell lines derived from murine mammary adenocarcinomas, as a gene expressed in cells capable of metastazing to the lung and bones, but only rarely in non-metastatic sublines or weakly metastatic to the lymph nodes (Christensen et al., 1998). Sema3E transcript was also detected in primary tumor samples from human breast cancers, but a correlation between Sema3E expression and metastatic activity has not been established (Christensen et al., 2005). We therefore analysed Sema3E mRNA abundance by quantitative real-time PCR in a panel of 67 breast tumor biopsies obtained from patients with different tumors.

Sema3E expression has been found at high level in breast cancer with axilary node involvement and in breast cancer with distant metastases.

### PlexinD1 and Sema3E are expressed in cancer cell lines

The above findings indicate that Sema3E and its receptor PlexinD1 are overexpressed in human breast tumors of high malignancy grade. We next studied whether Sema3E and PlexinD1 are also produced in different tumorigenic breast cancer cell lines. It has been reported that transcripts for *Sema3E* and *PlxnD1* are both expressed in human breast cancer cells MDA-MB-231 and MCF7 that derive from metastatic sites (Kigel et al., 2008). Using RT-PCR we showed that *Sema3E* and *PlxnD1* are also expressed in the mouse mammary adenocarcinoma cell line 4T1 (Aslakson et al., 1992), that has been shown to form primary tumors and distant metastasis in mouse model systems (Figure 2).

Expression was confirmed at the protein level by immunohistochemistry using anti-PlexinD1 and anti-Sema3E antibodies on MDA-MB-231, MCF-7 and 4T1 cells. Hek293T cells, which do not express endogenous Sema3E and PlexinD1 (Chauvet et al., 2007), were used as negative controls to show specificity of labeling (not shown). Finally, we also observed that Sema3E and PlexinD1 are expressed in U-87 MG human cell lines derived from malignant gliomas. See table 1.

**Table 1:**

| | Hek293T | MDA-MB-231 | MCF-7 | 4T1 | U-87 |
|---|---|---|---|---|---|
| PlexinD1 | - | + | + | + | + |
| Sema3E | - | + | + | + | + |

Together, these data indicate that aggressive tumor cells express Sema3E and its receptor PlexinD1, suggesting a possible autocrine function of Sema3E in cancer.

### Induction of cell death by inhibiting Sema3E in aggressive mouse and human cancer cell lines expressing Sema3E and PlexinD1, is mediated by PlexinD1.

To investigate whether inhibiting Sema3E in metastatic tumor cells affected their viability, the mouse mammary tumor cell line 4T1, developed by Miller and colleagues was used as a model of highly metastatic breast cancer cells with the capacity to metastasize efficiently to distant organs (Aslakson and Miller, 1992). To examine whether cell death associated to reduction of Sema3E level can be extended to human breast cancer cells, other cell lines were also used : MDA-MD-231 and MCF7 cells, two human breast cancer cell lines that derive from metastatic sites and co-express Sema3E and PlexinD1. shRNA against Sema3E was used to reduce Sema3E. Cell death was completely abolished in 4T1 cells which received Sema3E shRNAs concomitantly with a siRNA targeting PlexinD1 arguing that Sema3E production promotes 4T1 cancer cell survival by inhibiting PlexinD1-mediated cell death (Figure 2).

### SD1 blocks Sema3E/PlexinD1 interaction and Sema3E-mediated effects in vitro

Characteristic features of the PlexinD1 protein: the N-terminus sema domain is followed by three plexin-semaphorin-integrin (PSI) domains and three juxtamembrane IPT (Ig-like, plexins and transcription factors)-domains. The intracellular domains domain of plexins has two highly conserved regions (C1 and C2) that are similar to a GAP domain divided in two by a linker region. The conserved regions contain three arginine residues that are necessary for catalytic activity in GAPs.

To evaluate the role of Sema3E/PlexinD1 signaling in tumor cells, we generated a molecular tool capable of blocking Sema3E-induced PlexinD1 activation. This molecule, termed SD1, is composed of the immunoglobulin (Ig) k-chain secretion signal peptide, followed by the sema domain of mouse PlexinD1 protein (aa 61 to 531) fused in frame with six myc epitope tags (see scheme in Figure 3A).

We first set up co-precipitation experiments in transfected HEK293T cells to investigate whether the SD1 interacted with Sema3E ligand. As shown in Figure 3B, the SD1 polypeptide physically associated with Sema3E. In contrast, SD1 did not directly interact with PlexinD1 or its known co-receptors Neuropilin-1 and the vascular endothelial growth factor receptor 2, VEGFR2 (Chauvet et al., 2007; Bellon et al., 2010; data not shown). Next, we examined the displacement of Sema3E binding to Hek293T cells expressing PlexinD1 by different concentrations of SD1 (from 1-12 nM) present in conditioned medium of HEK293T cells transfected with the SD1 plasmid. SD1 was found to exert an antagonistic effect on Sema3E/PlexinD1 binding with an IC₅₀ of 5.05 nM (Figure 3C).

The results suggest that interfering with Sema3E/PlexinD1 may be used to trigger death of cancer cells.

### SD1 inhibits the development of primary tumors and metastasis in vivo

To find out if blocking the autocrine effect of Sema3E on tumor cells can modulate tumor development, we expressed a vector encoding SD1 or a control mock vector in the 4T1 cancer cells, which expresses endogenous Sema3E and PlexinD1 receptor, together with a myristoylated Cherry (mCherry) encoding plasmid. SD1 expression in the 4T1- SD1 stable transfectant was confirmed by Western blot analysis of the cell culture supernatant (Figure 4). Immunofluorescent staining of PlexinD1 and Sema3E on the 4T1-mock and 4T1-SD1 cell lines demonstrated that endogenous expression of PlexinD1 and Sema3E is not modified (data not shown). Immunolabeling of PlexinD1 and Sema3E on primary tumor sections derived from 4T1-mock and 4T1-SD1 cells injected subcutaneously into syngenic Balb/c mice demonstrated that expression of PlexinD1 and Sema3E appears normal in mCherry-positive 4T1 cells even in the presence of SD1. Expression of SD1 did not affect PlexinD1 or Sema3E protein levels in 4T1 cells kept *in vitro* or transplanted subcutaneously to form solid tumors in mice.

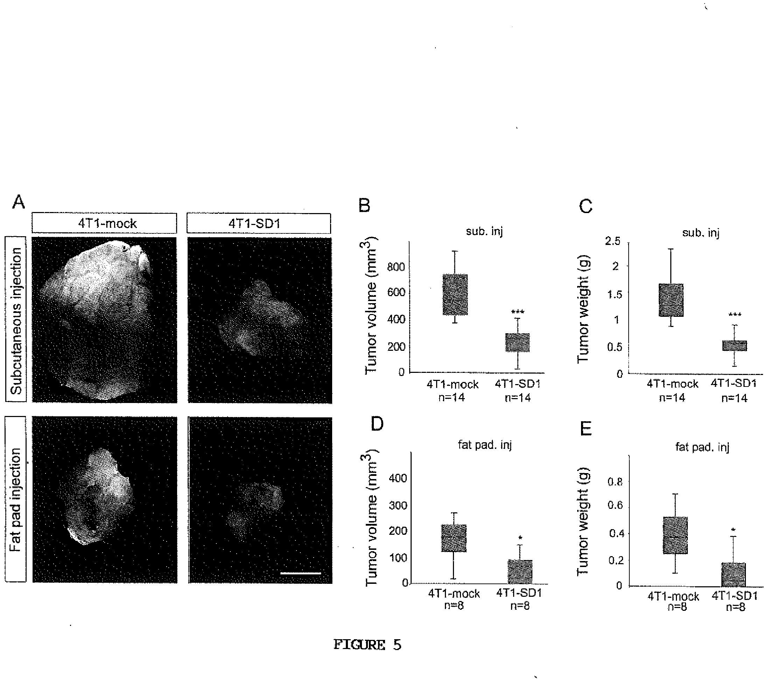

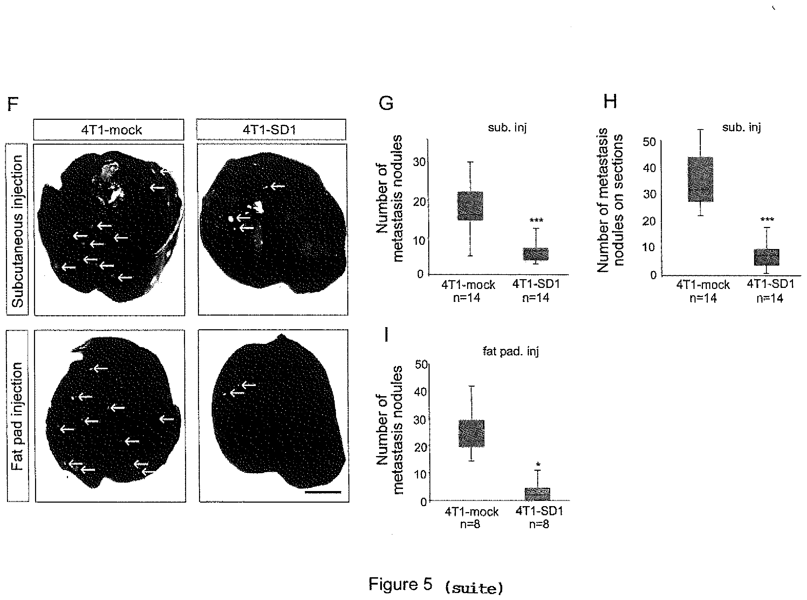

We next evaluated the effect of SD1 on tumor development and metastasic behavior in a syngenic breast cancer model in Balb/c mice, from which 4T1 cells were derived. In a first series of experiments, 4T1-SD1 or 4T1-mock cells were injected either subcutaneously or in their normal anatomical site, the mammary fat pad. The primary tumors formed from the injected cells were collected after 27 days. SD1 caused a significant reduction in the volume of the tumor (60% subcutaneous injection; 56% mammary fat pad injection; Figures 5A-5E), resulting in a decreased total tumor weight of 60% following subcutaneous injections (n=14/4T1-mock and n= 14/4T1-SD1) and of 56% following fat pad injections (n=8/4T1-mock and n= 8/4T1-SD1). We also harvested and inspected the lungs for metastatic tumor growth. A decrease in the number of metastatic nodules at the surface of the lungs was observed in animals injected with 4T1-SD1 cells, compared to those that had received control cells, both after subcutaneous (Figures 5F-5H) and fat pad transplantations (Figures 5F and 5I). To confirm this result, lungs were sectioned to quantify internal nodules. This analysis confirmed that the reduction in the number of visible superficial lung metastatic nodules of 4T1-SD1 injected mice, reflects a global decrease in the absolute number of lung metastasis (Figure 5H). Together, these results indicate that blocking endogenous Sema3E/PlexinD1 signaling inhibits tumor growth and progression.

These results suggest that interfering with Sema3E/PlexinD1 may be used to trigger death of cancer cells and counteract metastatic progression and dissemination *in vivo.*

In a second set of experiments, to understand whether SD1 suppresses the formation of metastatic nodules by direct anti-metastatic effect or indirectly via its limiting effect on primary tumor growth, mice were injected with 4T1-SD1 or 4T1-mock cells, and tissue samples were collected when tumors reached an average a same volume of 300 mm³ (4T1-mock/subcutaneous: 46 ± 4 dpi (day post-injection); 4T1-SD1 /subcutaneous: 60 ± 3 dpi; 4T1-mock/fat pad: 31 ± 3 dpi; 4T1-SD1 /fat pad: 50 ± 6 dpi; Figures 6A-6E). Comparison of lung metastasis in animals with tumors of similar size, indicated a reduction in the average number of metastatic nodules per lung in animals transplanted with 4T1-SD1 cells subcutaneously (n=6/4T1-mock and n= 6/4T1-SD1; Figures 6F and 6G) and in fat pad (n=5/4T1-mock and n= 5/4T1-SD1; Figures 6F and 6H), indicating a direct anti-metastatic effect of SD1.

### SD1 induces tumor cell death in vivo

To determine how SD1 regulates tumor growth and metastasis, we examined the concentration of tumor blood vessels in primary tumors formed by 4T1-SD1 or 4T1-mock cells by analyzing the pattern of expression of the endothelial marker PECAM-1 (platelet endothelial adhesion molecule-1). To this end, immunolabeling of PECAM-1 on primary tumor sections from 4T1-mock or 4T-SD1 cells injected subcutaneously into syngenic Balb/c mice was done and intensity of PECAM-1 immunofluorescence was reported on Figure 7.

Sema3E was characterized in different studies as a repulsive factor for endothelial cells (Gu et al., 2005; Toyofuku et al., 2007) and overexpression of Sema3E in cancer cells has an anti-angiogenic effect in some experimental tumors (Kigel et al., 2008; Roodink et al., 2008). Here we found that, blocking endogenous Sema3E function did not significantly affect the concentration of blood vessels when comparing tumors that developed from 4T1-SD1 cells with control tumors, despite a reduction in tumor size (Figure 7).

Tumor cell proliferation, as measured by Ki67 immunostaining, was also unchanged in tumors formed by 4T1-SD1 cells, compared to tumors that develop from 4T1-mock cells (Figure 8).

In order to show that the decrease noted in tumor growth and metastasis was due to the death induced by SD1, tumor sections were immunolabeled for cleaved Caspase-3 and Caspase-9, two markers of cell death.

Quantification of mCherry-positive 4T1 cells displaying cleaved Caspase-3 or Caspase-9 immunostaining indicated a ∼3 fold increase in tumor cell death in primary tumors formed by 4T1-SD1 cells compared to control cells (Figures 9A and 9B). To further test whether SD1 stimulated a death response exclusively in SD1-expressing tumor cells or could potentially exert a paracrine role, we co-injected subcutaneously mCherry-labelled 4T1-SD1 cells and GFP-labelled 4T1-mock cells (1:1 ratio) into syngenic mice. Analysis of cleaved Caspase-3 and Caspase-9 immunoreactivity in the developped tumors indicated that, when mixed with SD1-expressing cells, 4T1-mock cells died as much as 4T1-SD1 cells, indicating a non cell autonomous effect of the SD1 compound (Figures 9C and 9D). Interestingly, we noted that in contrast to tumor cells, stromal cells were not susceptible to SD1-induced death (Figures 9A-9D).

In order to determine whether the anti-cancer effect of SD1 involves pro-death PlexinD1 receptor signalling in tumor cells, the two cell lines, 4T1-SD1 and 4T1-mock, were stably transfected with a shRNA plasmid targeting PlexinD1 and the growth and metastatic behaviour of the four different cell lines (4T1-mock/shCont, 4T1-mock/shPlxnD1, 4T1-SD1/shCont and 4T1-SD1/shPlxnD1) in syngenic mice injected subcutaneously was investigated.

As shown in Figure 10, PlexinD1-deficient cells (4T1-mock/shPlxnD1) formed primary tumors that reached an average size comparable to that of tumors formed from injection of control cells (4T1-mock/shCont), and formed similar number of pulmonary metastases.

This effect was accompanied by a significant decrease of cleaved caspase-3 and caspase-9 immunostaining in tumors of 4T1-SD1/shPlxnD1 compared to 4T1-SD1/shCont tumors (Figure 11). Together, these data suggest that the inhibitory effect of SD1 on cancer progression involved engagement of pro-death signalling from the PlexinD1 dependence receptor in tumor cells, most probably through inhibition of autocrine Sema3E/PlexinD1 signalling.

### Blocking Sema3E autocrine activity induces PlexinD1-mediated death of tumor cells

We next used an *in vitro* assay to evaluate whether SD1 is directly capable of triggering death of tumor cells. We found that applying recombinant SD1 (present in conditioned medium from HEK293T cells transfected with a SD1 vector) to cultures of 4T1 cells induces cell death, measured by trypan blue-exclusion, in a dose dependent manner (Figure 12). Moreover, 4T1 cells also showed decreased viability when treated with SD1 purified from conditioned medium, as measured by calcein assay (Figure 13). Similarly, 4T1-SD1 cells, which proliferate at the same rate as 4T1-mock cells during the first 24-48h in culture, show increased death 72h after plating, possibly as a result of SD1 accumulation in the culture medium (Figure 14). Interestingly, transfecting 4T1-SD1 cells with a siRNA sequence that downregulates PlexinD1 expression (Chauvet et al., 2007) significantly decreased this cell-death effect as measured by trypan blue-exclusion (Figure 15), active caspase-3 immunostaining (Figure 16) or MTT assay (Figure 17). Control experiments with 4T1-mock cells transfected with PlexinD1 siRNA revealed no noticeable impact on cell death/survival (Figures 15, 16 and 17). Thus, SD1 triggers cancer cell death in a PlexinD1 dependent manner.

The above results suggest that autocrine signaling by Sema3E via PlexinD1 is important for 4T1 cancer cell survival.

To further confirm the importance of Sema3E for cell viability, we knocked down Sema3E expression using two different short hairpin (sh) RNAs (shRNA1 and shRNA2; Figure 18) in 4T1 cells and monitored cell death. We found that reducing Sema3E level using shRNA1 induced death of 4T1 cells, an effect that was reversed by applying exogenous Sema3E protein in the cell culture medium (Figures 18-21). Similar to what observed when Sema3E signaling was blocked using SD1, this death effect required PlexinD1 function since it was completely abolished in cells co-transfected with a siRNA to PlexinD1 (Figure 19). The same results were obtained using Sema3E shRNA2 (data not shown). Taken together, these observations argue that Sema3E production promotes 4T1 cancer cell survival by inhibiting PlexinD1-mediated cell death.

Finally, to test whether it is possible to generalize these results to other cancer cell types, we applied 15 nM SD1 (present in conditioned medium from HEK293T cells transfected with SD1 plasmid) to cultures of the human breast cancer cells MDA-MB-231 and MCF-7, which co-express Sema3E and PlexinD1. This treatment leads to a reduction in cell survival as measured by calcein assay (Figure 22). The same effect was obtained after electroporation of MDA-MB-231 and MCF-7 with a shRNA that decreases human Sema3E expression (Figures 23-24). Thus, disruption of Sema3E/PlexinD1 autocrine signaling inhibits survival of human breast cancer cells.

### PlexinD1 is a dependence receptor

The finding that death of 4T1 cells induced by expression of SD1 or Sema3E shRNA is rescued by inhibiting PlexinD1 function is consistent with the idea that PlexinD1 is a novel member of the 'dependence receptor' family (Goldschneider and Mehlen, 2010). Such receptors are able to initiate two types of signaling: in the presence of ligand, a "positive" signaling is transduced, whereas in the absence of ligand interaction, they induce an apoptotic cell death. To directly assay the death activity of PlexinD1, we transiently expressed full length human PlexinD1 or mouse PlexinD1 in HEK293T cells. PlexinD1 from both species caused increased cell death measured by trypan blue-exclusion (Figure 25), quantification of cells stained with anti-active caspase-3 antibody (Figure 26) or MTT (Figure 27). We therefore used the human PlexinD1 in all subsequent experiments. To exclude the possibility that death induction could be caused by a general toxic effect of PlexinD1 autoactivation, a mutant receptor lacking the catalytic activity of R-Ras GTPase Activating Protein (GAP) (human PlexinD1-RA) was expressed in HEK293T cells. This mutant fails to induce inhibition of neuronal axon growth in response to Sema3E (Uesugi et al., 2009; data not shown), however, it displays a similar death activity than wild-type PlexinD1 receptor (Figures 25-27), indicating that PlexinD1 triggers cell death independently of its endogenous R-Ras GAP activity. Finally, we demonstrated that the death effect of PlexinD1 is caspase-dependent as it was blocked by inhibitors of caspase-3 (z-DEVD-fmk) and capspase-9 (z-LEHD-fmk), but not caspase-8 (z-IETD-fmk) inhibitor (Figures 28 and 29).

We next examined whether the presence of Sema3E affects the death activity of PlexinD1. As shown in Figure 30, PlexinD1-triggered cell death was inhibited in a dose dependent manner by adding exogenous Sema3E ligand. We also found that exogenous Sema3E blocked the death effect induced by the mutant PlexinD1-RA receptor (Figure 30). PlexinD1 protein level was unchanged in HEK293T cells transfected with PlexinD1 vectors and treated with Sema3E compared to untreated conditions, suggesting that change in PlexinD1 expression is unlikely to account for this rescue from cell death. Similarly, HEK293T cells expressing PlexinD1 could be saved from cell death by co-transfection with a vector encoding Sema3E (Figure 31). However, no change in PlexinD1-induced cell death was observed following transfection with a vector encoding another class 3 semaphorin, Sema3C, which does not directly bind to PlexinD1 (Figure 31). Finally, we also tested the effect of a soluble form of the class 4 semaphorin, Sema4A, which has been reported to bind to PlexinD1 with an affinity constant ∼7 times lower than that of Sema3E (Toyofuku et al., 2007), although other investigators have failed to confirm Sema4A binding to PlexinD1 (Choi et al., 2008). We found that co-expressing Sema4A together with PlexinD1 in HEK-293T cells was ineffective to save the cells from death (Figure 31). Taken together, these data show that PlexinD1 act as a dependence receptor that triggers cell death in the absence of its Sema3E ligand. PlexinD1 from both species caused increased cell death and this death is reversed by addition of the ligand Sema3E.

### PlexinD1 intracellular domain is cleaved by caspase

To better understand the mechanism underlying PlexinD1 pro-apoptotic activity, we further investigated whether the executioner caspase-3 cleaves the intracellular domain of PlexinD1, as reported for a number of other dependence receptors (Goldschneider and Mehlen, 2010). The intracellular region of hPlexinD1 (aa 1293 to 1925) was translated *in vitro* and the product was incubated with active caspase-3. Figure 32 shows that the intracellular domain of PlexinD1 is cleaved by caspase-3, resulting in five protein products that migrate at apparent molecular weight of 16, 24, 30, 37 and 50 kDa, suggesting the presence of at least two cleavage sites (Figure 32). These sites were mapped at two consensus caspase-3 recognition motifs, at aspartic acids (D) residues 1471 and 1624, that are highly conserved throughout vertebrate PlexinD1 receptors (Figures 33). Mutation of both aspartic acid residues to asparagine (N) within the intracellular domain of PlexinD1 completely suppressed caspase-3 cleavage and the appearance of the five protein fragments (Figure 32).

To examine the functional importance of the cleavage of PlexinD1, we expressed the intracellular domains of wild-type PlexinD1 (PlexinD1-IC), PlexinD1-IC D1471N mutant, PlexinD1-IC D1624N mutant and PlexinD1-IC D1471N/D1624N double mutant in HEK293T cells. Like full length PlexinD1, PlexinD1-IC induced cell death and the mutations on single caspase cleavage site did not significantly change cell death levels (Figure 34). In contrast, mutations at both caspase sites reduced the death activity of PlexinD1 intracellular domain (Figure 34). Moreover, we showed that expression of the protein fragment located between D1471 and D1624 was sufficient to cause death of HEK293T cells at the same level than that induced by PlexinD1-IC (Figure 35). Together, these results suggest that caspase cleavage of PlexinD1 may release a pro-death fragment (PlexinD1 1471-1624).

### REFERENCES

Unified nomenclature for the semaphorins/collapsins. Semaphorin Nomenclature Committee. (1999), Cell 97, 551-552.
Angeloni, D. et al. (2004), J Biol Chem 279, 3726-3732.
Aslakson, C.J., and Miller, F.R. (1992), Cancer Res 52, 1399-1405.
Bellon, A. et al. (2010),Neuron 66, 205-219.
Bork, P.et al. (1999),Trends Biochem Sci 24, 261-263.
Capparuccia, L., and Tamagnone, L., J Cell Sci 122, 1723-1736.
Chauvet, S. et al. (2007), Neuron 56, 807-822.
Choi, Y.I. et al. (2008), Immunity 29, 888-898.
Christensen, C. et al. (2005), Cancer Res 65, 6167-6177.
Christensen, C.R.L. et al. (1998), Cancer Research 58, 1238-1244.
Chung, L. et al. (2007), Development 134, 2935-2945.
Gherardi, E. et al. (2004), Curr Opin Struct Biol 14, 669-678.
Gherardi, E. et al. (2003), Proc Natl Acad Sci U S A 100, 12039-12044.
Gitler, A.D. et al. (2004), Dev Cell 7, 107-116.
Goldschneider, D., and Mehlen, P (2010), Oncogene 29, 1865-1882.
Gu, C. et al. (2005), Science 307, 265-268.
Herman JG et al. (2004), Proc Am Assoc Cancer Res 45: 617.
Herman, J.G. and Meadows, G.G. (2007), International Journal of Oncology 30, 1231-1238.
Kanda, T. et al., G.G. (2007). J Cell Biochem 101, 1329-1337.
Kigel, B. et al. (2008), PLoS One 3, e3287.
Koppel, A.M. et al. (1997), Neuron 19, 531-537.
Mann, F. et al. (2007), Prog Neurobiol 82, 57-79.
Moriya, J. et al. (2010), Circ Res 106, 391-398.
Oinuma, I. et al. (2004), Science 305, 862-865.
Pecho-Vrieseling, E. et al. (2009), Nature 459, 842-846.
Rohm, B. et al. (2000a), Mech Dev 93, 95-104.
Rohm, B. et al. (2000b), FEBS Lett 486, 68-72.
Roodink, I. et al. (2008), Am J Pathol 173, 1873-1881.
Roodink, I. et al. (2009), BMC Cancer 9, 297.
Sakurai, A. et al. (2010), Mol Cell Biol. Epub
Takahashi, T. et al. (1999), Cell 99, 59-69.
Tamagnone, L. et al. (1999), Cell 99, 71-80.
Toyofuku, T. et al. (2007), EMBO J 26, 1373-1384.
Toyofuku, T. et al. (2008), Dev Biol 321, 251-262.
Uesugi, K. et al. (2009), J Biol Chem 284, 6743-6751.
van der Zwaag, B. et al. (2002), Dev Dyn 225, 336-343.
Zhang, Y. et al. (2009), Dev Biol 325, 82-93.

## Claims

1. A molecule antagonist of the Sema3E/PlexinD1 interaction for use in the treatment of primary cancer bearing PlexinD1 and with Sema3E expression, wherein the molecule specifically binds to PlexinD1 or Sema3E and blocks Sema3E/PlexinD1 interaction, and wherein the molecule is an antagonist polypeptide comprising or consisting of a PlexinD1 domain that specifically binds to Sema3E or an antagonist Sema3E domain that specifically binds to PlexinD1, or an antagonist antibody that specifically binds to Sema3E or an antagonist antibody that specifically binds to PlexinD1.

2. The molecule for the use of claim 1, wherein the antagonist polypeptide comprises or consists of a PlexinD1 sema domain.

3. The molecule for the use of claim 1, wherein the antagonist polypeptide comprises or consists of the amino acid sequence SEQ ID NO: 1 or an amino acid sequence as encoded by SEQ ID NO: 2.

4. The molecule for the use of claim 1, wherein the antagonist polypeptide comprises or consists of a Sema3E amino acid sequence, which is the binding region for PlexinD1.

5. The molecule for the use of any one of claims 1 to 4, wherein the antagonist polypeptide is a chimeric polypeptide comprising said PlexinD1 or Sema3E amino acid sequence fused to a heterologous amino acid sequence, preferably a Fc domain.

6. The molecule for the use of claim 1, wherein the molecule is an antagonist antibody against PlexinD1 sema domain.

7. The molecule for the use of claim 1, wherein the antibody is an antagonist antibody against the sema binding sequence of Sema3E.

8. The molecule for the use of any one of the preceding claims, wherein the molecule is used in the treatment of primary cancer and metastasis with Sema3E expression.

9. The molecule for the use of any one of claims 1 to 7, wherein the cancer is breast cancer, prostate cancer, melanoma or glioblastoma.

10. The molecule for the use of claim 9, wherein the cancer is breast cancer with axillary node involvement or distant metastases.

11. The molecule for the use of any one of claims 1 to 7, wherein the cancer is a lung cancer with Sema3E expression.

12. The molecule for the use of any one of the preceding claims, wherein the molecule induces inhibition of primary cancer cell development, or induces PlexinD1-mediated death of tumor cells or tumor cell apoptosis.

13. In vitro method for selecting a compound for the prevention or the treatment of cancer expressing PlexinD1 and Sema3E wherein said method comprises the following steps of :
a) having a medium containing Sema3E, or a fragment thereof, and a PlexinD1 receptor, or a fragment thereof, wherein said Sema3E, or a fragment thereof, and said PlexinD1 receptor, or a fragment thereof, are able to specifically interact together to form a binding pair,
b) contacting said medium with the compound to be tested ;
c) measuring the inhibition of the interaction between Sema3E, or a fragment thereof, and said PlexinD1 receptor, or a fragment thereof, and
d) selecting said compound if the measuring in step c) demonstrates a significant inhibition of the interaction between Sema3E, or a fragment thereof, and PlexinD1 receptor, or a fragment thereof, in presence of said compound .

14. An *in vitro* method for selecting a patient as suitable to be treated with a molecule according to any one of claims 1 to 12, comprising detecting the presence of a cancer with PlexinD1 and Sema3E expression, which detection is made in a patient having a primary tumor from a biopsy or a blood or serum sample of said patient containing primary tumors cells, said method comprising the step of measuring of the PlexinD1 and Sema3E expression levels in said biopsy or a blood or serum sample, and the step of selecting the patient expressing PlexinD1 and Sema3E as suitable to be treated with said molecule.

## Patentansprüche

1. Molekül-Antagonist der Sema3E/PlexinD1-Wechselwirkung zur Verwendung bei der Behandlung von primärem Krebs, der PlexinD1 trägt und Sema3E-Expression aufweist, wobei das Molekül spezifisch an PlexinD1 oder Sema3E bindet und die Sema3E/PlexinD1-Wechselwirkung blockiert, und wobei das Molekül ein antagonistisches Polypeptid ist, das eine PlexinD1-Domäne, die spezifisch an Sema3E bindet, oder eine antagonistische Sema3E-Domäne, die spezifisch an PlexinD1 bindet, umfasst oder daraus besteht, oder ein antagonistischer Antikörper, der spezifisch an Sema3E bindet, oder ein antagonistischer Antikörper, der spezifisch an PlexinD1 bindet.

2. Molekül zur Verwendung nach Anspruch 1, wobei das antagonistische Polypeptid eine PlexinD1-Sema-Domäne umfasst oder daraus besteht.

3. Molekül zur Verwendung nach Anspruch 1, wobei das antagonistische Polypeptid die Aminosäuresequenz SEQ ID Nr: 1 oder eine Aminosäuresequenz, wie sie von SEQ ID Nr: 2 kodiert wird, umfasst oder daraus besteht.

4. Molekül zur Verwendung nach Anspruch 1, wobei das antagonistische Polypeptid eine Sema3E-Aminosäuresequenz umfasst oder daraus besteht, die die Bindungsregion für PlexinD1 ist.

5. Molekül zur Verwendung nach einem der Ansprüche 1 bis 4, wobei das antagonistische Polypeptid ein chimäres Polypeptid ist, das die PlexinD1- oder Sema3E-Aminosäuresequenz, fusioniert mit einer heterologen Aminosäuresequenz, vorzugsweise einer Fc-Domäne, umfasst.

6. Molekül zur Verwendung nach Anspruch 1, wobei das Molekül ein antagonistischer Antikörper gegen die Sema-Domäne von PlexinD1 ist.

7. Molekül zur Verwendung nach Anspruch 1, wobei der Antikörper ein antagonistischer Antikörper gegen die Sema-Bindesequenz von Sema3E ist.

8. Molekül zur Verwendung nach einem der vorhergehenden Ansprüche, wobei das Molekül zur Behandlung von primärem Krebs und Metastasen mit Sema3E-Expression verwendet wird.

9. Molekül zur Verwendung nach einem der Ansprüche 1 bis 7, wobei der Krebs Brustkrebs, Prostatakrebs, Melanom oder Glioblastom ist.

10. Molekül zur Verwendung nach Anspruch 9, wobei der Krebs Brustkrebs mit Axillarknotenbeteiligung oder Fernmetastasen ist.

11. Das Molekül zur Verwendung nach einem der Ansprüche 1 bis 7, wobei der Krebs ein Lungenkrebs mit Sema3E-Expression ist.

12. Molekül zur Verwendung nach einem der vorhergehenden Ansprüche, wobei das Molekül eine Hemmung der Entwicklung von primären Krebszellen induziert oder den PlexinD1-vermittelten Tod von Tumorzellen oder die Apoptose von Tumorzellen induziert.

13. In vitro-Verfahren zur Auswahl einer Verbindung zur Prävention oder Behandlung von Krebs, der PlexinD1 undSema3E exprimiert, wobei das Verfahren die folgenden Schritte umfasst:
a) Bereitstellen eines Mediums, das Sema3E oder ein Fragment davon und einen PlexinD1-Rezeptor oder ein Fragment davon enthält, wobei das Sema3E oder ein Fragment davon und der PlexinD1-Rezeptor oder ein Fragment davon spezifisch miteinander wechselwirken können, um ein Bindungspaar zu bilden,
b) Kontaktieren des Mediums mit der zu prüfenden Verbindung;
c) Messen der Hemmung der Wechselwirkung zwischen Sema3E oder einem Fragment davon und dem PlexinD1-Rezeptor oder einem Fragment davon, und
d) Auswählen der Verbindung, wenn das Messen in Schritt c) eine signifikante Hemmung der Wechselwirkung zwischen Sema3E oder einem Fragment davon und dem PlexinD1-Rezeptor oder einem Fragment davon bei Vorhandensein der Verbindung zeigt.

14. *In-vitro*-Verfahren zum Auswählen eines Patienten, der für eine Behandlung mit einem Molekül nach einem der Ansprüche 1 bis 12 geeignet ist, umfassend das Nachweisen des Vorhandenseins eines Krebses mit PlexinD1- und Sema3E-Expression, wobei der Nachweis in einem Patienten mit einem Primärtumor anhand einer Biopsie oder einer Blut- oder Serumprobe des Patienten, die Primärtumorzellen enthält, erfolgt, wobei das Verfahren den Schritt des Messens des PlexinD1- und Sema3E-Expressionsniveaus in der Biopsie oder einer Blut- oder Serumprobe und den Schritt des Auswählens des Patienten, der PlexinD1 und Sema3E exprimiert, als für eine Behandlung mit dem Molekül geeignet umfasst.

## Revendications

1. Une molécule antagoniste de l'interaction Sema3E/PlexineD1 pour une utilisation dans le traitement d'un cancer primaire porteur de PlexineD1 et ayant une expression de Sema3E, dans lequel la molécule se lie spécifiquement à PlexineD1 ou à Sema3E et bloque l'interaction Sema3E/PlexineD1, et dans laquelle la molécule est un polypeptide antagoniste comprenant ou constitué par un domaine de PlexineD1 qui se lie spécifiquement à Sema3E ou un domaine de Sema3E antagoniste qui se lie spécifiquement à PlexineD1, ou un anticorps antagoniste qui se lie spécifiquement à Sema3E ou un anticorps antagoniste qui se lie spécifiquement à PlexineD1.

2. Molécule pour l'utilisation selon la revendication 1, dans laquelle le polypeptide antagoniste comprend ou est constitué par un domaine sema de PlexineD1.

3. Molécule pour l'utilisation selon la revendication 1, dans laquelle le polypeptide antagoniste comprend ou est constitué par la séquence d'acides aminés SED ID NO : 1 ou une séquence d'acides aminés telle que codée par SEQ ID NO : 2.

4. Molécule pour l'utilisation selon la revendication 1, dans laquelle le polypeptide antagoniste comprend ou est constitué par une séquence d'acides aminés de Sema3E, qui est la région de liaison pour PlexineD1.

5. Molécule pour l'utilisation selon l'une quelconque des revendications 1 à 4, dans laquelle le polypeptide antagoniste est un polypeptide chimérique comprenant ladite séquence d'acides aminés de PlexineD1 ou Sema3E fusionnée avec une séquence d'acides aminés hétérologue, de préférence un domaine Fc.

6. Molécule pour l'utilisation selon la revendication 1, dans laquelle la molécule est un anticorps antagoniste contre un domaine sema de PlexineD1.

7. Molécule pour l'utilisation selon la revendication 1, dans laquelle l'anticorps est un anticorps antagoniste contre la séquence de liaison sema de Sema3E.

8. Molécule pour l'utilisation selon l'une quelconque des revendications précédentes, dans laquelle la molécule est utilisée dans le traitement d'un cancer primaire et d'une métastase ayant une expression de Sema3E

9. Molécule pour l'utilisation selon l'une quelconque des revendications 1 à 7, dans laquelle le cancer est un cancer du sein, un cancer de la prostate, un mélanome ou un glioblastome.

10. Molécule pour l'utilisation selon la revendication 9, dans laquelle le cancer est un cancer du sein avec l'implication d'un ganglion axillaire ou des métastases distantes.

11. Molécule pour l'utilisation selon l'une quelconque des revendications 1 à 7, dans laquelle le cancer est un cancer du poumon avec une expression de Sema3E.

12. Molécule pour l'utilisation selon l'une quelconque des revendications précédentes, dans laquelle la molécule induit l'inhibition du développement de cellules de cancer primaire, ou induit la mort médiée par PlexineD1 de cellules tumorales ou une apoptose de cellules tumorales.

13. Procédé in vitro pour la sélection d'un composé pour la prévention ou le traitement d'un cancer exprimant PlexineD1 et Sema3E, dans lequel ledit procédé comprend les étapes suivantes :
a) avoir un milieu contenant Sema3E, ou un fragment de celui-ci, et un récepteur PlexineD1, ou un fragment de celui-ci, dans lequel ledit Sema3E, ou un fragment de celui-ci, et ledit récepteur PlexineD1, ou un fragment de celui-ci, sont capables d'interagir spécifiquement ensemble pour former une paire de liaison,
b) mettre en contact ledit milieu avec le composé à mettre à l'essai ;
c) mesurer l'inhibition de l'interaction entre Sema3E, ou un fragment de celui-ci, et ledit récepteur PlexineD1, ou un fragment de celui-ci, et
d) sélectionner ledit composé si la mesure à l'étape c) démontre une inhibition significative de l'interaction entre Sema3E, ou un fragment de celui-ci, et le récepteur PlexineD1, ou un fragment de celui-ci, en présence dudit composé.

14. Procédé *in vitro* pour sélectionner un patient comme approprié pour être traité avec une molécule selon l'une quelconque des revendications 1 à 12, comprenant la détection de la présence d'un cancer avec une expression de PlexineD1 et Sema3E, la détection étant réalisée chez un patient ayant une tumeur primaire à partir d'une biopsie ou un échantillon de sang ou de sérum dudit patient contenant des cellules de tumeur primaire, ledit procédé comprenant l'étape consistant à mesurer les taux d'expression de PlexineD1 et de Sema3E dans ladite biopsie ou ledit échantillon de sang ou de sérum, et l'étape consistant à sélectionner le patient exprimant PlexineD1 ou Sema3E comme approprié pour être traité avec ladite molécule.
